# EUROPEAN PATENT SPECIFICATION

(11) **EP 4 144 350 B1**
(45) Date of publication and mention of the grant of the patent: **07.05.2025**
(21) Application number: 21194373.3
(22) Date of filing: 01.09.2021
(51) Int. Cl.: A61K 31/345, A61K 31/496, A61P 9/10

(54) **TREATMENT OR PREVENTION OF VASCULAR MALFORMATION**
BEHANDLUNG ODER VORBEUGUNG VON GEFÄSSMISSBILDUNG
TRAITEMENT OU PRÉVENTION DE MALFORMATION VASCULAIRE

(43) Date of publication of application: 08.03.2023
(73) Proprietor: Universitätsmedizin Greifswald, 17475 Greifswald (DE)
(72) Inventor: Rath, Matthias, 17475 Greifswald (DE); Felbor, Ute, 17475 Greifswald (DE); Schwefel, Konrad, 17475 Greifswald (DE)
(74) Representative: Altmann Stößel Dick Patentanwälte PartG mbB

(56) References cited:
- WO-A1-2016/053938
- WO-A1-2019/115472
- CN-A- 110 693 886
- US-A1- 2019 298 733

## Description

The present invention relates in a first aspect to a compound of formula (I) or a pharmaceutically acceptable salt thereof for use in treatment or prevention of a vascular malformation. A second aspect of the invention is direct to a pharmaceutical preparation comprising a compound of formula (I) or pharmaceutically acceptable salt thereof. In a third aspect, the invention is directed to a method for identifying a subject profiting from treatment with a compound of formula (I) or pharmaceutically acceptable salt thereof.

Vascular malformation, especially cerebral cavernous malformations (CCM), also known as cavernous haemangiomas or cavernomas, refer to a benign vascular malformation consisting of a cluster of altered blood vessels, i.e. CCMs are low-flow vascular lesions prone to cause severe haemorrhage-associated neurological complications.

CCMs account for 10 to 15 % of all vascular lesions in the central nervous system [1]. CCMs are often asymptomatic but can cause significant neurological complications when they bleed. They can cause bleeding even at low pressure, which either remains undetected, or manifests itself in the form of headaches, paralysis or seizures. Cavernomas can occur in the brain as well as in the spinal cord. They can be congenital but can also develop in the course of life.

Therapeutic options are still limited. Neurosurgical resection can be indicated in some cases, but there is a substantial risk of early postoperative morbidity for CCMs in the brainstem or other eloquent areas [2].

CCMs can be found in a sporadic and autosomal dominant form. The latter is associated with pathogenic germline variants in either *CCM1* (aka *KRIT1*)*, CCM2* or *CCM3* (aka *PDCD10*)*.* Patients with familial CCM often present at a younger age with multiple cavernomas [3]. The number of vascular lesions can even increase over time. Genetic and immunohistochemical analyses of human cavernoma tissues have established a Knudsonian two-hit model of familial CCM disease [4]. Only when a somatic mutation inactivates the corresponding wild-type allele in a brain endothelial cell (EC) of a germline mutation carrier, CCM formation is initiated. With sophisticated transgenic *Ccm3* mouse models, recent studies have shed light on the stages of CCM genesis and demonstrated that clonal expansion of mutant ECs and recruitment of wild-type or heterozygous ECs trigger early CCM formation and later lesion growth, respectively [5, 6]. Recently, clonal dominance of mutant ECs and resistance to staurosporine-induced apoptosis in human umbilical vein and brain microvascular ECs following CRISPR/Cas9-mediated *CCM3* gene disruption were shown [7].

Compounds for treatment of cancer are known, for example, WO 2016/053938 A1 discloses NSC59984 for treating or preventing cancer.

It is however commonly known that CCMs are no malignant tumors. Nevertheless, some features of CCM disease are reminiscent of tumourigenesis. Based on the latest insights into CCM pathogenesis, novel therapeutic approaches have been proposed but have not been all investigated so far: (1) Targeted eradication or inhibition of CCM progenitor ECs that have acquired a second, somatic mutation; (2) blocking the clonal expansion of mutant ECs; and (3) inhibiting the recruitment and incorporation of neighbouring heterozygous or wild-type ECs [8]. For example, blocking the clonal expansion of mutant ECs using active pharmaceutical compounds has not been investigated so far. Even if, for example, a substance called Ponatinib has been analyzed in a mouse model of CCM, it has not been clarified whether this influences the clonal expansion of mutant ECs [30]. A rho kinase inhibitor selective for ROCK2 for treating CCM in a mammal is described in US /2019298733 A1. Compounds of a specific formula for CCM treatment are also disclosed in CN 110 693 886 A, as well as polycomb inhibitors in WO 2019/115472 A1. Taken together, therapeutic options are still limited.

Thus, there is an ongoing need to provide further new therapeutics for treating vascular malformations. The object of the present invention was thus the provision of agents suitable to treat or prevent a vascular malformation.

### 1^{st} aspect - compound of formula (I) for use in treatment or prevention of a vascular malformation

The problem was solved by a compound of formula (I) 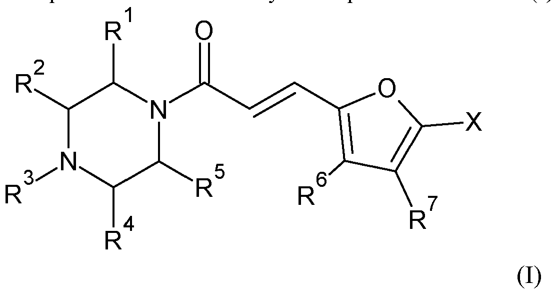 wherein
- R¹ R², R⁴ and R⁵: are independently either a hydrogen atom or a straight or branched C1 to C3 alkyl group;
- R³: is a hydrogen atom or a straight or branched C1 to C5 alkyl group;
- R⁶, R⁷: are independently either a hydrogen atom or a straight or branched C1 to C3 alkyl group; and
- X: is selected from the group consisting of -F, -Cl, -Br, -I, -CN, -NO₂, -OR⁸, -SR⁸, -S(=O)R⁸, -S(=O)₂R⁵, -NHS(=O)₂R⁸, -C(=O)R⁸, -OC(=O)R⁸, -CO₂R⁸ -OCO₂R⁸, -CH(R⁸)₂, -N(R⁸)₂ -C(=O)N(R⁸)₂, -OC(=O)N(R⁸)₂, -NHC(=O)NH(R⁸)₂, -NHC(=O)R⁸, -NHC(=O)OR⁸, -C(OH) (R⁸)₂, and -C(NH₂)(R⁸)₂, wherein R⁸ is a hydrogen atom or a C1 to C3 alkyl group;
or a pharmaceutically acceptable salt thereof for use in treatment or prevention of a vascular malformation.

### Regarding the compound of formula (I):

Preferably, at least two, preferably at least three, of R¹, R², R⁴ and R⁵ are hydrogen atoms and the remaining residue of R¹, R², R⁴ and R⁵ is a straight or branched C1 to C3 alkyl group.

Preferably, R³ is a straight or branched C1 to C5 alkyl group, preferably a straight or branched C1 to C3 alkyl group, more preferably an ethyl or methyl group, more preferably a methyl group.

Preferably, at least one of R⁶, R⁷ is a hydrogen atom, preferably R⁶ and R⁷ are both hydrogen atoms.

Preferably, X is selected from the group consisting of -CN, -NO₂, -C(=O)R⁸, -CO₂R⁸, -S(=O)R⁸ and -S(=O)₂R⁸, preferably from the group consisting of -CN, -NO₂, -C(=O)R⁸ -CO₂R⁸, wherein R⁸ is a hydrogen atom or a C1 to C3 alkyl group, wherein X is more preferably a -NO₂ group.

A preferred embodiment of the invention is directed to the compound of formula (I) or pharmaceutically acceptable salt thereof being NSC59984 of formula (Ia) or a pharmaceutically acceptable salt thereof for use in treatment or prevention of vascular malformation

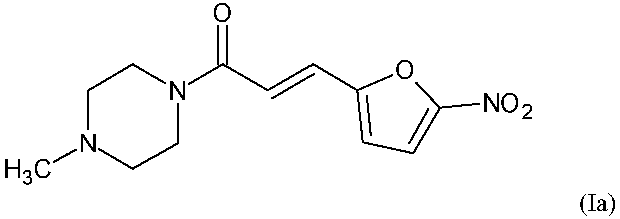

NSC59984 is (E)-1-(4-methylpiperazin-1-yl)-3-(5-nitrofuran-2yl)prop-2-en-1-one (CAS: 803647-40-7). For NSC59984 it was so far only shown that the compound inhibits tumour cell growth [9].

Based on screening of an apoptosis compound library, it was found that compounds of formula (I) or their pharmaceutically acceptable salts induce cell cycle arrest in *CCM3*^{*-*/*-*} cells and efficiently blocks their expansion in co-culture with *CCM3*^{*+*/*+*} ECs. That is, compounds of formula (I) or their pharmaceutically acceptable salts are considered to be effective in treatment or prevention of vascular malformations. In line with the concept that CCMs harbour tumour-like features, a striking clonal expansion of CCM3-deficient endothelial cells in co-culture with wild-type cells was demonstrated. The activator of p53 signalling - a compound of formula (I), especially a compound of formula (Ia), NSC59984 - proved to be a potent inhibitor of CCM3-deficient cell proliferation. The small molecule induced upregulation of *CDKN1A* (p21) and *GADD45A* and led to cell cycle arrest in *CCM3* mutant endothelial cells. The positive effect of treatment with a compound of formula (I), especially a compound of formula (Ia), NSC59984, was verified with patient-specific *CCM1*^{*-*/*-*} blood outgrowth endothelial cells.

The vascular malformation preferably has a genetic component. A "vascular malformation having a genetic component means a hereditary vascular malformation caused by a pathogenic germline variant in a disease-related gene and/or a sporadic vascular malformation caused by one or more somatic mutation(s). Preferably, the vascular malformation is selected from the group consisting of hereditary hemorrhagic telangiectasia, capillary malformation-arteriovenous malformation, glomuvenous malformations, infantile hemangioma, and cerebral cavernous malformation, wherein the vascular malformation is preferably cerebral cavernous malformation (CCM).

Several preferred embodiments are related to the compound of formula (I), preferably NSC59984 of formula (Ia), the compound not being present in a salt form, for use in treatment or prevention of vascular malformation.

As used herein the term "pharmaceutically acceptable salts" refers to addition salts of the compounds with acids or bases that form anions or cations, which are, preferably, non-toxic to the recipient thereof. Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate salts. Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others.

The compound of formula (I) or pharmaceutically acceptable salt thereof as described in the section related to the first aspect can be used for a pharmaceutical preparation - this use not being covered by the claims.

A pharmaceutical preparation may have a solid form more preferably a solid form selected from the group of tablet, granulate, powder, capsule and mixtures of two or more of these forms.

Alternatively, the pharmaceutical preparation may have a liquid form preferably a liquid form selected from solution and emulsion.

The term "pharmaceutical composition", as used herein, may related to a mixture of compounds comprising at least NSC59984 or pharmaceutically acceptable salt thereof as specified herein and, preferably, at least one carrier. NSC59984 and/or a pharmaceutically acceptable salt thereof, which may be comprised in the pharmaceutical composition, are described herein above. The pharmaceutical composition may have any consistency deemed appropriate by the skilled person. The pharmaceutical composition may be a solid composition, e.g. a tablet or a powder, a semisolid composition, e.g. a gel, or, , a liquid, e.g. a solution or an emulsion.

The pharmaceutical composition may comprise a carrier. The carrier(s) preferably may be acceptable in the sense of being compatible with the other ingredients of the pharmaceutical composition and being not deleterious to a potential recipient thereof. The carrier(s) preferably may be selected so as not to affect the biological activity of the pharmaceutical composition. Preferably, the pharmaceutical composition may be sterile, more preferably a sterile solution, most preferably a sterile solution for injection. The carrier is selected by the skilled person such as to achieve the consistency intended and may be, for example, a gel or, preferably, a liquid, more preferably an aqueous liquid. Examples of such carriers are distilled water, physiological saline, Ringer's solutions, dextrose solution, phosphate -buffered saline solution, and Hank's solution. The carrier may include one or more solvents or other ingredients increasing solubility of the compounds comprised in the pharmaceutical composition. Further examples of liquid carriers are syrup, oil such as peanut oil and olive oil, water, emulsions, various types of wetting agents, sterile solutions and the like. Suitable carriers comprise those mentioned above and others well known in the art. As is understood by the skilled person, the pharmaceutical composition may comprise one or more further compounds; preferably, such additional compounds are selected so as to not affect the biological activity of the pharmaceutical composition, in particular of the active NSC59984 and/or a pharmaceutically acceptable salt thereof, is acceptable in the sense of being compatible with the other ingredients of the pharmaceutical composition and being not deleterious to a potential recipient thereof.

Preferably, the carrier may be a pharmaceutically acceptable carrier. In addition, the pharmaceutical composition or formulation may also include other carriers, adjuvants, stabilizers and/or other compounds deemed appropriate by the skilled person, e.g. for galenic purposes. As referred to herein, the NSC59984 or pharmaceutically acceptable salt thereof as specified, may be the "active compound" of the preparation, although "further active compounds", which are referred to under this term, may be present. Preferably, the active compound and the further active compound, i.e. preferably the active compounds, may bepharmaceutically active compounds. Specific pharmaceutical compositions may be prepared in a manner well known in the pharmaceutical art and comprise at least one active compound referred to herein above, preferably in admixture or otherwise associated with at least one pharmaceutically acceptable carrier or diluent. For making those specific pharmaceutical compositions, the active compound(s) would usually be mixed with a carrier or the diluent. The resulting formulations may be adapted to the mode of administration, i.e. in the forms of tablets, capsules, suppositories, solutions, suspensions or the like. Dosage recommendations shall be indicated in the prescriber's or user's instructions in order to anticipate dose adjustments depending on the considered recipient.

The pharmaceutical composition may bepreferably, administered systemically or topically. Suitable routes of administration conventionally used for drug administration are oral, topical, intravenous, or parenteral administration as well as inhalation. Preferably, administration may be oral or parenteral. Other modes of administration may be specified elsewhere herein. Moreover, the pharmaceutical composition may be administered in combination with other further active compounds either in a common pharmaceutical composition or as separated pharmaceutical compositions wherein said separated pharmaceutical compositions may be provided in form of a kit of parts.

The pharmaceutical composition may be preferably, administered in conventional dosage forms prepared by combining the active compound with standard pharmaceutical carriers according to conventional procedures. These procedures may involve mixing or dissolving the ingredients as appropriate to obtain the desired preparation. It will be appreciated that the form and character of the pharmaceutically acceptable carrier or diluent may be dictated by the amount of active ingredient with which it is to be combined, the route of administration and other well-known variables. Similarly, the carrier or diluent may include time delay material well known in the art, such as glyceryl mono-stearate or glyceryl distearate alone or with a wax. A therapeutically effective dose refers to an amount of the active compound to be used in a pharmaceutical composition of the present invention which provides the effect referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen may be be determined by the attending physician and other clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can may be, for example, in the range of 1 µg to 1000 mg; however, doses below or above this exemplary range may be envisioned, especially considering the aforementioned factors. Generally, the regimen as a regular administration of the pharmaceutical composition should be in the range of 1 µg to 100 mg units per day. If the regimen is a continuous infusion, it should also be in the range of 1 µg to 1 mg units per kilogram of body weight per minute, respectively. Depending on the subject and the mode of administration, the quantity of substance administration may vary over a wide range to provide from about 0.01 mg per kg body mass to about 100 mg per kg body mass. The pharmaceutical compositions and formulations referred to herein may be administered preferably administered more than one time, more preferably on a continuous basis and/or in regular intervals. Preferably, administration may be adjusted to maintain an effective concentration in the body of a subject for the time period intended.

### 2^{rd} aspect - method for identifying a subject profiting from treatment

A second aspect of the invention is related to a method for identifying a subject profiting from treatment with a compound of formula (I), preferably NSC59984 of formula (Ia), or pharmaceutically acceptable salt thereof as described in the section related to the first aspect, wherein the method for identifying a subject profiting from treatment with a compound of formula (I), or pharmaceutically acceptable salt comprises
(a) determining degree of vascular malformation in a sample of said subject;
(b) treating the sample with compound of formula (I) or pharmaceutically acceptable salt thereof as described in the section related to the first aspect;
(c) determining degree of vascular malformation in a sample of said subject after (b);
(d) identifying a subject profiting from treatment with a compound of formula (I) or pharmaceutically acceptable salt thereof as described in the section related to the first aspect in case the degree of vascular malformation is found to be reduced in (c).

The "degree" of vascular malformation is determined based on one or more parameters selected from the group consisting of number of malformations, size/size growth of malformations, frequency of bleeding events (especially in CCM) and proliferation of mutant endothelial cells (ECs).

The method for identifying a subject profiting from treatment with a compound of formula (I), or pharmaceutically acceptable salt thereof is preferably an *in vitro* method, which is preferably based on proliferation of mutant endothelial cells (ECs).

A further aspect of the invention is a method for treatment or prevention of vascular malformation in a subject suffering therefrom, comprising
(A) administering a compound of formula (I) or pharmaceutically acceptable salt thereof as described in the section related to the first aspect to said subject, and
(B) thereby treating or preventing vascular malformation.

The terms "treating" and "treatment" refer to an amelioration of the diseases or disorders referred to herein or the symptoms accompanied therewith to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20% at least 50% at least 60%, at least 70%, at least 80%, or at least 90% of the subjects of a given cohort or population. Preferably, treating vascular malformation is reducing vascular malformation burden in a subject. As will be understood by the skilled person, effectiveness of treatment of e.g. vascular malformation is dependent on a variety of factors including, e.g. vascular malformation stage and type. Preferably, treating causes effector cells to undergo quiescence and/or growth arrest.

The term "preventing" refers to retaining health with respect to the diseases or disorders referred to herein for a certain period of time in a subject. It will be understood that the said period of time may be dependent on the amount of the compound which has been administered and individual factors of the subject. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The term "subject", as used herein, relates to a mammal. More preferably, the subject is a human. Preferably, the subject is known to suffer from vascular malformation or the subject is suspected to suffer from vascular malformation. "Suspected to suffer from vascular malformation", also in the sense of prevention, means that a subject has been identified based on, for example, a genetic disposition. Patients with suspected hereditary vascular malformations are offered genetic germline testing. Identification of a pathogenic variant enables predictive testing for further family members. Carriers of a pathogenic germline variant who are at risk of developing vascular malformations can be identified by predictive genetic testing.

The present invention is further illustrated by the following set of embodiments and combinations of embodiments resulting from the dependencies and back-references as indicated. In particular, it is noted that in each instance where a range of embodiments is mentioned, for example in the context of a term such as " any one of embodiments (1) to (4) ", every embodiment in this range is meant to be explicitly disclosed for the skilled person, i.e. the wording of this term is to be understood by the skilled person as being synonymous to "any one of embodiments (1), (2), (3), and (4)". Further, it is explicitly noted that the following set of embodiments is not the set of claims determining the extent of protection, but represents a suitably structured part of the description directed to general and preferred aspects of the present invention.

### Embodiment (1): A compound of formula (I)

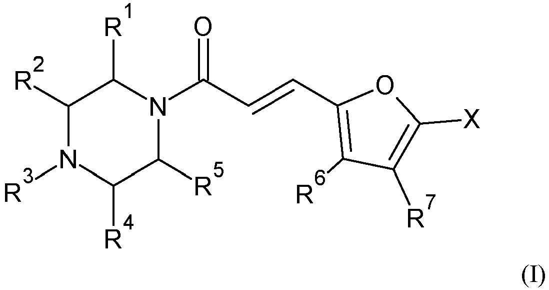

wherein
- R¹, R², R⁴ and R⁵: are independently either a hydrogen atom or a straight or branched C1 to C3 alkyl group;
- R³: is a hydrogen atom or a straight or branched C1 to C5 alkyl group; R⁶, R⁷ are independently either a hydrogen atom or a straight or branched C1 to C3 alkyl group; and
- X: is selected from the group consisting of -F, -Cl, -Br, -I, -CN, -NO₂, -OR⁸, -SR⁸, -S(=O)R⁸, -S(=O)₂R⁵, -NHS( =O)₂R⁸, -C(=O)R⁸, -OC(=O)R⁸, -CO₂R⁸, -OCO₂R⁸ -CH(R⁸)₂, -N(R⁸)₂ -C(=O)N(R⁸)₂, -OC(=O)N(R⁸)₂, -NHC(=O)NH(R⁸)₂, -NHC(=O)R⁸, -NHC(=O)OR⁸, -C(OH) (R⁸)₂, and -C(NH₂)(R⁸)₂, wherein R⁸ is a hydrogen atom or a C1 to C3 alkyl group;
or a pharmaceutically acceptable salt thereof for use in treatment or prevention of a vascular malformation.

Embodiment (2): The compound of formula (I) or pharmaceutically acceptable salt thereof for use according to embodiment (1), wherein at least two, preferably at least three, of R¹, R², R⁴ and R⁵ are hydrogen atoms and the remaining residue of R¹, R², R⁴ and R⁵ is a straight or branched C1 to C3 alkyl group.

Embodiment (3): The compound of formula (I) or pharmaceutically acceptable salt thereof for use according to embodiment (1) or (2), wherein R³ is a straight or branched C1 to C5 alkyl group, preferably a straight or branched C1 to C3 alkyl group, more preferably an ethyl or methyl group, more preferably a methyl group.

Embodiment (4): The compound of formula (I) or pharmaceutically acceptable salt thereof for use according to any one of embodiments (1) to (3), wherein at least one of R⁶, R⁷ is a hydrogen atom, preferably R⁶ and R⁷ are both hydrogen atoms.

Embodiment (5): The compound of formula (I) or pharmaceutically acceptable salt thereof for use according to any one of embodiments (1) to (4), wherein X is selected from the group consisting of -CN, -NO₂, -C(=O)R⁸ -CO₂R⁸, -S(=O)R⁸ and -S(=O)₂R⁸, preferably from the group consisting of -CN, -NO₂, -C(=O)R⁸ -CO₂R⁸, wherein R⁸ is a hydrogen atom or a C1 to C3 alkyl group, wherein X is more preferably a -NO₂ group.

Embodiment (6): The compound of formula (I) or pharmaceutically acceptable salt thereof for use according to any one of embodiments (1) to (5), wherein the compound is NSC59984 of formula (Ia)

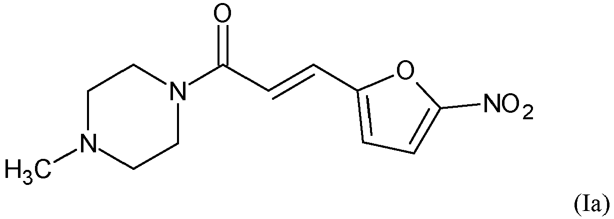

Embodiment (7): The compound or pharmaceutically acceptable salt thereof according to any one of embodiments (1) to (6) for use in treatment or prevention of vascular malformation, wherein the vascular malformation has a genetic component.

Embodiment (8): The compound or pharmaceutically acceptable salt thereof according to any one of embodiments (1) to (7) for use in treatment or prevention of vascular malformation, wherein the vascular malformation is selected from the group consisting of hereditary hemorrhagic telangiectasia, capillary malformation-arteriovenous malformation, glomuvenous malformations, infantile hemangioma, and cerebral cavernous malformation, wherein the vascular malformation is preferably cerebral cavernous malformation (CCM).

Embodiment (9): The compound according to any one of embodiments (1) to (8) for use in treatment or prevention of vascular malformation.

Embodiment (10): A method for identifying a subject profiting from treatment with a compound of formula (I) or pharmaceutically acceptable salt thereof according to any one of embodiments (1) to (9), comprising
(a) determining degree of vascular malformation in a sample of said subject;
(b) treating the sample with compound of formula (I) or pharmaceutically acceptable salt thereof according to any one of embodiments 1 to 9;
(c) determining degree of vascular malformation in a sample of said subject after (b);
(d) identifying a subject profiting from treatment with compound of formula (I) or pharmaceutically acceptable salt thereof according to any one of embodiments 1 to 9 in case the degree of vascular malformation is found to be reduced in (c).

Embodiment (11): The method of embodiment (10) which is an *in vitro* method, which is preferably based on proliferation of mutant endothelial cells (ECs).

Embodiment (12): A method for treatment or prevention of vascular malformation in a subject suffering therefrom, comprising
(A) administering a compound of formula (I) or pharmaceutically acceptable salt thereof as described in any one of embodiments (1) to (9) to said subject, and
(B) thereby treating or preventing vascular malformation.

The present invention is further illustrated by the following reference examples, comparative examples, and examples.

### Examples

### MATERIALS AND METHODS

### Cell culture

Constitutive immortalised human umbilical vein endothelial cells (CI-huVECs, InSCREENeX, Braunschweig, Germany) were cultured at 37 °C and 5 % CO₂ in endothelial cell growth medium (ECGM, PromoCell, Heidelberg, Germany) supplemented with 10 % foetal calf serum (FCS, Thermo Fisher Scientific, Waltham, MA, USA).

Clonally expanded CI-huVEC lines with biallelic loss-of-function variants in the first coding exon of *CCM3* have been described before or were established according to a published CRISPR/Cas9 genome editing protocol [7]. Blood outgrowth endothelial cells (BOECs) were cultured in EGM-2 medium (Lonza, Basel, Switzerland) supplemented with 18 % FCS. With the approval of the local ethics committee (University Medicine Greifswald, Germany; No.: BB 047/14a), *CCM1*^{*-*/*-*} BOECs had been established previously from *CCM1*^{+/-} BOECs of a *CCM1* mutation carrier with CRISPR/Cas9 genome editing [17]. Mutant and wild-type ECs were co-cultured in 96-well plates under standard culture conditions with the indicated mutant-to-wild-type ratios. 2500 cells were seeded per well.

An apoptosis compound library with 189 small molecules that target apoptosis, survival and proliferation was used in a high throughput screening approach (HY-L003[HY-LD-000001651]; MedChem Express, Monmouth Junction, NJ, USA). Co-cultures were treated with 10 µM of each substrate for six days. Compounds were added 5 h and 3 days after cell seeding.

NSC59984 (IUPAC: (2E)-1-(4-Methyl-1-piperazinyl)-3-(5-nitro-2-furyl)-2-propen-1-one, HY19726, MedChem Express), was used for individual experiments with the indicated concentrations.

DMSO (A994, Carl Roth, Karlsruhe, Germany)-treatment served as control.

If not stated otherwise CI-huVECs were used for experiments in passage 14-16 or 31-33, respectively. BOECs were used in passage 15-18.

### T7EI cleavage assay and amplicon deep sequencing

The fraction of mutant alleles in co-culture were analysed by amplicon deep sequencing or T7EI cleavage assay. In brief, genomic target regions were first amplified by PCR. For T7E1 cleavage assays, heteroduplexes were formed and digested with T7EI enzyme (New England Biolabs, Frankfurt am Main, Germany) [7, 36]. Cleavage products were visualised by gel electrophoresis and analysed with the Image Lab software (v6.0, Bio-Rad, Hercules, CA, USA). For NGS analysis, sequencing adapters and individual barcodes were introduced in a second round of PCR [7]. Sequencing libraries were pooled and sequenced on a MiSeq instrument with 2 x 150 cycles (Illumina, San Diego, CA, USA). The data were analysed with the SeqNext software (JSI Medical Systems, Ettenheim, Germany).

### Clonogenic assay and cell cycle analysis

Plating efficiencies were determined as follows: *CCM3*^{*+*/*+*} and *CCM3*^{*-*/*-*} CI-huVEC were seeded in 6-well-plates with either 100 or 250 cells per well. Colonies fixed and stained after eight days with a solution of glutaraldehyde (G6257, Sigma-Aldrich, final concentration 6 %) and crystal violet (HT901, Sigma-Aldrich, final concentration 0.5 %). The plating efficiency was defined as the ratio between the number of colonies and the number of plated cells. A cell cycle analysis kit (MAK344, Sigma-Aldrich) was used to determine cell cycle profiles by flowcytometry (LSRII Flow Cytometer System, BD Biosciences, San Jose, CA, USA). One day after plating, cells were synchronised for 24 h with ECGM supplemented with 0.1 % FCS. Prior to FACS analysis, cells were incubated with ECGM + 10 % FCS for another 24 h or treated with 10 µM NSC59984 for 72 h. Data were analysed with FlowJo v10.7.1 (BD Biosciences).

### Proliferation assay, caspase-3, caspase-8, and caspase-9 activity assay

The proliferation rate of mutant and wild-type ECs in monoculture was quantified with the CyQuant^{®} Cell Proliferation Assay Kit (C7026, Thermo Fisher Scientific). To discriminate the proliferation rates of *CCM3*^{+/+} and *CCM3*^{-/-} CI-huVEC in co-culture, the CyQuant^{®} Cell Proliferation Assay results were combined with mutant allele frequencies that had been determined by amplicon deep sequencing. To analyse their sensitivity to apoptosis, mutant and wild-type ECs were seeded in 96-well plates with 15.000 cells per well, and treated with staurosporine (S440, Sigma-Aldrich, St. Louis, MO, USA) 24 h later. The Caspase-3 DEVD-R110 Fluorometric HTS Assay Kit (30009, Biotium, Fremont, CA, USA), the Cell Meter^{™} Caspase 8 Activity Apoptosis Assay Kit (22798, AAT Bioquest, Sunnyvale, CA, USA), and the Cell Meter^{™} Caspase 9 Activity Apoptosis Assay Kit (22799, AAT Bioquest) were used following the manufacturer's instructions.

### RNA isolation and RNA sequencing

RNA was extracted using the PeqGold TriFast reagent (Peqlab-VWR, Radnor, PA, USA) and purified using the Direct-zol RNA MiniPrep Plus Kit (Zymo Research, Irvine, CA, USA). A Qubit 4.0 (Thermo Fisher Scientific) and the Qubit RNA BR Assay Kit (Q10211, Thermo Fisher Scientific) were used to measure RNA concentrations. RNA sample integrity was controlled on a 2100 Bioanalyzer using the RNA 6000 Nano Kit (Agilent, Santa Clara, CA, USA). A QIAseq Targeted Human Apoptosis and Cell Death RNA Panel (RHS-002Z, QIAGEN, Hilden, Germany) was used to prepare RNA sequencing libraries according to the manufacturer's instructions. After quality control with the High Sensitivity DNA Kit on a 2100 Bioanalyzer instrument (Agilent), the pooled libraries were sequenced on a MiSeq (Illumina) with 1x151 cycles. Differential gene expression of RNA sequencing data was carried out using the QIAseq Targeted RNA Primary and Secondary Analysis Tool (QIAGEN).

### Apoptosis antibody-array, protein extraction and Western Blot analyses

The Human Apoptosis Antibody Array - Membrane Kit (ab134001, Abcam, Cambridge, UK) was used according to the manufacturer's instructions to analyse the expression of apoptosis related proteins in *CCM3*^{*+*/*+*} and *CCM3-*/*-* CI-huVEC after 24 h of treatment with 0.05 µM staurosporine. 600 µg of total protein was incubated for each membrane, and chemiluminescence signals were documented with a ChemiDoc XRS+ (Bio-Rad, Hercules, California, USA) imager. Densitometry data were obtained using the ImageLab software (v6.0, Bio-Rad). Solubilisation of proteins extracted with PeqGold TriFast reagent (Peqlab-VWR) was performed in buffer containing 8 M Urea, 2 M Thio-Urea, and 20 mM Tris.

### Differentiation of hiPSCs to hBMECs and co-culture

Fluorescently tagged human induced pluripotent stem cells (Coriell Institute, Camden, NJ, USA) were maintained at 37 °C and 5 % CO2 in Essential 8 Flex medium (Thermo Fisher Scientific, Waltham, MA, USA) on plates coated with growth factor reduced matrigel (Corning Inc., Corning, NY, USA). Cells were passaged with 0.5 mM EDTA (Thermo Fisher Scientific). The following endogenously tagged cell lines as part of the Allen Cell Collection (Coriell Institute) were used: AICS-0036-006:WTC-mEGFP-Safe harbor locus (AAVS1)-cl6 and AICS-0054-091:WTC-mTagRFPT-CAAX-Safe harbor locus (AAVS1)-cl91.

*CCM3*^{*+*/*+*} AICS-0054 hiPSCs, *CCM3*^{*+*/*+*} and *CCM3*^{*-*/*-*} AICS-0036 hiPSCS were differentiated into human brain microvacular endothelial-like cells (hBMECs). Briefly, 158,000 cells were seeded in Essential 8 Flex medium containing 10 µM Y-27632 on growth factor reduced Matrigel-coated 6-well plates. After 24 h, differentiation was initiated by substitution of Essential 8 Flex with Essential 6 medium (Thermo Fisher Scientific). Medium was changed daily for 4 days. On day 4, medium was switched to Human Endothelial Serum Free Medium (hESFM, Thermo Fisher Scientific) supplemented with FGF-2 (Miltenyi Biotec, Bergisch Gladbach, Germany), retinoic acid (Sigma-Aldrich), insulin (Sigma-Aldrich), holo-transferrin (Sigma-Aldrich), and sodium selenite (Sigma-Aldrich). After 48 h, cells were replated on 24-well plates coated with collagen IV from human placenta (Sigma-Aldrich) and human plasma fibronectin (Sigma-Aldrich). 24 h later, medium was changed to hESFM supplemented with insulin, holo-transferrin, and sodium selenite until confluency was reached. Differentiated hBMECs were passaged and expanded in EndoGRO-MV medium (Merck) supplemented with 1 ng/ml FGF-2. To observe clonal expansion of mutant hBMECs, *CCM3*^{*+*/*+*} AICS-0054 hBMECs and *CCM3*^{+/+} or *CCM3*^{-/-} AICS-0036 hBMECs were mixed in a 9:1 ratio and cultured for 6 days. On day 6, wells were fixed with 4 % paraformaldehyde (PFA) or replated onto 96-well plates. On day 12, remaining wells were fixed and Hoechst 33342 (Thermo Fisher Scientific) staining was performed for subsequent fluorescent imaging analysis with the EVOS FL imaging system. The ratio of EGFP-tagged AICS-0036 cells compared to all cells was determined using the ImageJ software. For the experiments, differentiated hBMECs in passage 3-5 were used.

### Differentiation of hiPSCs to vascular organoids

hiPSCs were differentiated into vascular organoids according to the protocol of Wimmer and colleagues [39]. Briefly, 800,000 hiPSCs were seeded in ultra-low attachment 6-well plates (Corning) in aggregation medium for 2 days until aggregates had formed. On day 0, mesodermal induction was initiated with CHIR99021 (Tocris Bioscience, Bristol, United Kingdom) and BMP-4 (Miltenyi Biotec) in N2B27 medium. Supplementation of VEGF-A (PeproTech, Inc., Rocky Hill, NJ, USA) and forskolin (Sigma-Aldrich) on day 3 induced vascular differentiation. On day 5, vascular aggregates were embedded in a collagen I-Matrigel matrix. VEGF-A, FGF-2 (Miltenyi Biotec), and FBS (Thermo Fisher Scientific) enriched StemPro-34 medium (Thermo Fisher Scientific) was added for vessel sprouting. Vascular network extraction was performed on day 10.

### Integrin antibody-array, ITGB4 qPCR and staining, FACS sorting

The β-Integrin-mediated Cell Adhesion Array Kit (ECM534, Sigma-Aldrich) was used according to the manufacturer's instructions to quantify cell surface expression of β₁, β₂, β₃, β₄, α_{V}β₅, and α₅β₁ integrins of *CCM3*^{*+*/*+*} and *CCM3*^{*-*/*-*} CI-huVECs. Fluorescence intensity was measured with the Qubit 4 Fluorometer (Thermo Fisher Scientific). For immunofluorescent imaging, CI-huVECS were cultured on 96-well plates until confluency was reached. Mouse anti-integrin β4 (ITGB4) antibody (1:100, MAB2059Z, Merck, Darmstadt, Germany) was added to the culture medium and cells were incubated for 1 h at 37 °C and 5 % CO₂. Cells were washed 3 times with culture medium and incubated for 1 h at 37 °C and 5 % CO₂ with goat anti-mouse, Alexa Fluor 488 secondary antibody (1:200, A-11029, Thermo Fisher Scientific). Subsequently, cells were washed 3 times in culture medium and imaged with the EVOS FL imaging system (Thermo Fisher Scientific). For qPCR analysis, mRNA was transcribed into cDNA by using the First Strand cDNA Synthesis Kit (Thermo Fisher Scientific). SYBR Green-based qPCR analysis was performed on a Roche Light Cycler 480 instrument (Roche, Mannheim, Germany) to validate a deregulated gene expression of *ITGB4.* The gene *RPLP0* served as an endogenous control. The following primer pairs purchased from Integrated DNA Technologies were used: ITGB4 - 5'-CTACTACGAGAAGCTTCACAC-3' and 5'-GACCCAGTCCTCGTCTTCTG-3'; RPLP0 - 5'-TCGACAATGGCAGCATCTAC-3' and 5'-ATCCGTCTCCACAGACAAGG-3'. For FACS sorting of co-cultured CI-huVECs, 1 million cells were seeded on T75-flasks in a 2:5 ratio of mutant to wild-type cells and cultured for 2 days. Cells were then dissociated with Accutase and stained with mouse anti-integrin β4 antibody (1:200) and goat anti-mouse, Alexa Fluor 488 secondary antibody (1:200). Cells were resuspended in PBS and FACS sorted on the BD FACSAria III Cell Sorter (BD Biosciences, Franklin Lakes, NJ, USA). The RNA of ITGB4 high and ITGB4 low fractions were extracted in TRI Reagent for fluid samples (Sigma-Aldrich). To confirm purity of sorted fractions, Sanger sequencing of extracted DNA and ICE analysis (Synthego, Redwood City, CA, USA) were performed that is used to determine the spectrum and frequency of CRISPR edits.

### Genome-wide RNA sequencing

For genome-wide RNA-seq, isolated RNA was concentrated with the RNA Clean & Concentrator-5 Kit (Zymo Research) prior to quality assessment. RNA-seq library preparation and mRNA sequencing via polyA selection on the Illumina NovaSeq platform (Illumina) with 2x150 cycles were performed by GENEWIZ (Leipzig, Germany). Reads were trimmed to remove adapter sequences using Trimmomatic v.0.36 and mapped to the Homo sapiens GRCh37 genome with STAR aligner v.2.5.2b. The software featureCounts from the Subread package v.1.5.2 was used to extract unique gene hit counts.

### Statistical analysis

The GraphPad Prism software (v.8.0.1, GraphPad Software, LA Jolla, CA, USA) was used for statistical analysis. Unless stated otherwise, all data are presented as mean and standard deviation (SD). For analyses of two or more groups, multiple t test, one- and two-tailed Student's t test as well as one- and two-way ANOVA were used. Dunnett's or Šidák's corrections were applied for multiple comparisons. The DESeq2 method was used to normalize targeted RNA sequencing data data. P-values or adjusted p-values <0.05 were regarded as statistically significant.

### Results

### CCM3 inactivation in ECs induced deregulation of TNF superfamily ligands and their receptors

To compare the growth and survival characteristics of wild-type and mutant ECs, first the extent to which *CCM3*^{*+*/*+*} and *CCM3*^{*-*/*-*} CI-huVECs could form colonies from single cells was analysed. A significantly higher clonogenic capacity of mutant CI-huVECs was observed, resulting in a more than 20 % increased plating efficiency (Fig. 1, Fig. 2). Despite an enhanced potential to survive, CI-huVECs in which the *CCM3* gene had been disrupted by CRISPR/Cas9 ribonucleoprotein (RNP) transfection did not display significantly different proliferation rates (Fig. 3). Next, apoptotic cell death was stimulated in *CCM3*^{*+*/*+*} and *CCM3*^{*-*/*-*} CI-huVECs with staurosporine and measured the activity of caspase-3, which is a major downstream effector caspase in apoptosis. In line with previous findings caspase-3 activity was significantly lower in mutant CI-huVECs already after two hours, and still after 24 hours (Fig. 4).

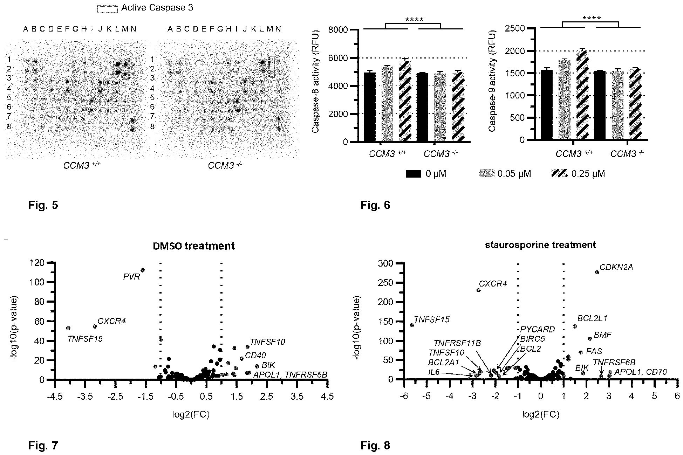

Measuring the relative expression of 43 apoptosis-related proteins verified the reduced fraction of active caspase-3 but revealed no other significantly up- or downregulated targets in *CCM3*^{*-*/*-*}CI-huVECs (Fig. 5). Caspase-3 is proteolytically activated by caspases of the intrinsic and extrinsic apoptosis pathway. As the apoptosis array only detected the active form of caspase 3, specific fluorogenic indicators were used to measure the activities of caspase-8 (extrinsic pathway) and caspase-9 (intrinsic pathway), which likewise were reduced in mutant ECs after staurosporine treatment (Fig. 6).

Targeted RNA sequencing verified deregulation of both pathways (Fig. 7, Fig. 8). Members of the Bcl-2 family (e.g. *BCL2A1, BCL2, BCL2L1,* and *BMF*) and genes that encode for members of the tumour necrosis factor (TNF) receptor superfamily or their ligands were found to be differentially expressed. Most importantly, reduction of the cytokine TL1A (alias *TNFSF15* or vascular endothelial growth inhibitor) and upregulation of the decoy receptor 3 (DcR3, alias *TNFRSF6B)* were seen. TL1A is known to be predominantly produced in ECs, in which it suppresses proliferation and angiogenesis [11]. DcR3 functions as a soluble decoy receptor of TL1A, but also of FasL and LIGHT. By binding and neutralising these TNF superfamily members, DcR3 can inhibit apoptosis, induce proliferation and migration of venous ECs, and exhibits angiogenic activity in vivo [12]. Interestingly, DcR3 is overexpressed in various malignant tumours, and TL1A has been reported to have potent anticancer activity [13, 14]. The present RNA sequencing analysis suggested two mechanisms by which *CCM3* mutant CI-huVECs can acquire survival advantage: (1) derepression of proliferation by TL1A downregulation and (2) induction of proliferation and anti-apoptosis by upregulation of DcR3.

### Co-culture with CCM3^{+/}⁺ cells stimulated enhanced proliferation of CCM3^{-/}⁻ ECs

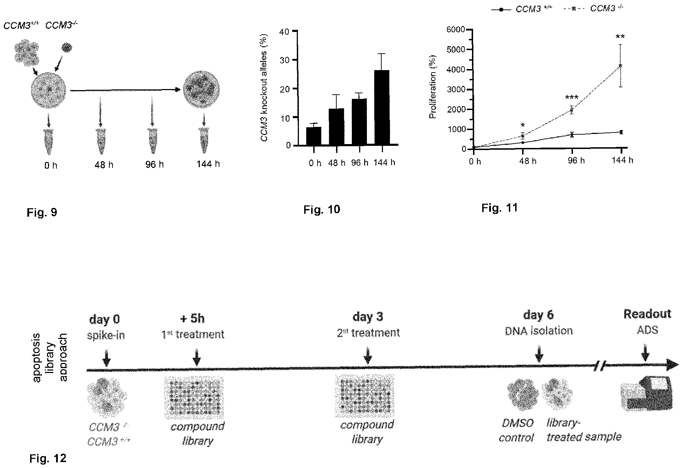

To better reflect the mosaic endothelial pattern in CCM tissues, *CCM3*^{*-*/*-*} CI-huVECs were co-cultured with *CCM3*^{*+*/*+*} ECs in a 1:9 ratio for six days (Fig. 9). To track the clonal expansion of mutant ECs, the fraction of knockout alleles at different time points was measured (Fig. 10). Remarkably, proliferation rates of mutant ECs were significantly increased in co-culture (4124 % vs 793 % after 144 h, Fig. 11). Furthermore, mutant ECs were resistant to replicative senescence. Next, the question was whether the proliferative advantage of mutant ECs in co-culture with wild-type ECs depends on cell-cell-interactions or can also be triggered by cytokine signalling. Thus, the effect of conditioned medium on the proliferation of *CCM3*^{*-*/*-*} and *CCM3*^{*+*/*+*} CI-huVECs was studied. As no effect was seen in any of the different conditions, cytokine release does not seem to be sufficient to induce clonal expansion of mutant ECs in co-culture.

### NSC59984 efficiently blocked clonal expansion of CCM3^{-/}⁻ ECs in co-culture

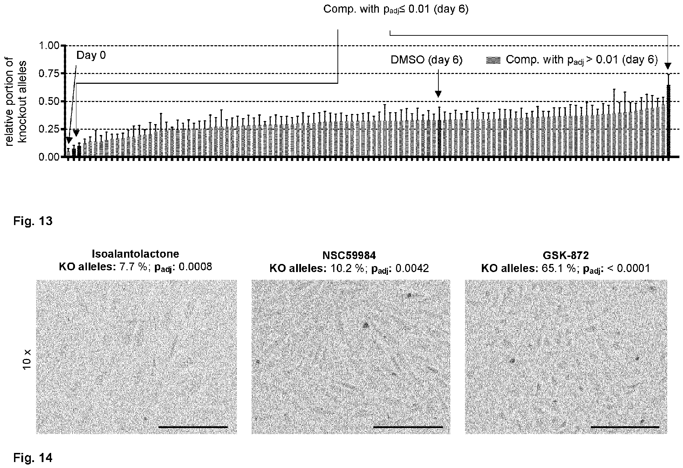

To identify a substance that selectively induces apoptosis or growth arrest in mutant ECs a library of 189 small compounds known to modulate apoptosis, survival, and proliferation was tested. The relative portions of knockout alleles were determined after six days of treatment by amplicon deep sequencing (Fig. 12). As expected, abnormal mutant cell proliferation and an increase of the knockout allele frequency to 33.5 % were observed in DMSO-treated co-culture controls. Interestingly, three compounds significantly modulated mutant cell expansion (Fig. 13). Knockout allele frequencies were markedly reduced by isoalantolactone (7.7 %, p_{adj} = 0.0008) and the p53 reactivator NSC59984 (10.2 %, p_{adj} = 0.0042). In contrast, the RIP kinase 3-inhibitor GSK-872, which interferes with an alternative mode of regulated cell death, called necroptosis, but can also trigger caspase-8-dependent apoptosis [16], even boosted *CCM3*^{*-*/*-*} CI-huVEC expansion in co-culture (65.1 %, p_{adj} < 0.0001). While treatment with isoalantolactone induced an abnormal endothelial phenotype and significant cell death, NSC59984 had minimal effects on EC morphology and induced apoptosis at a tolerable level. On the other hand, GSK-872 increased cell viability at low concentrations, induced moderate caspase-3 activation, and led to a more compact EC morphology (Fig. 14, Fig. 15, Fig. 16).

Altogether, the compound library screening identified NSC59984 as a promising candidate for CCM treatment since it blocked mutant cell expansion in co-culture and had a favourable cytotoxic profile.

### NSC59984 worked as a cytostatic drug and inhibits clonal expansion of CCM1 mutant ECs

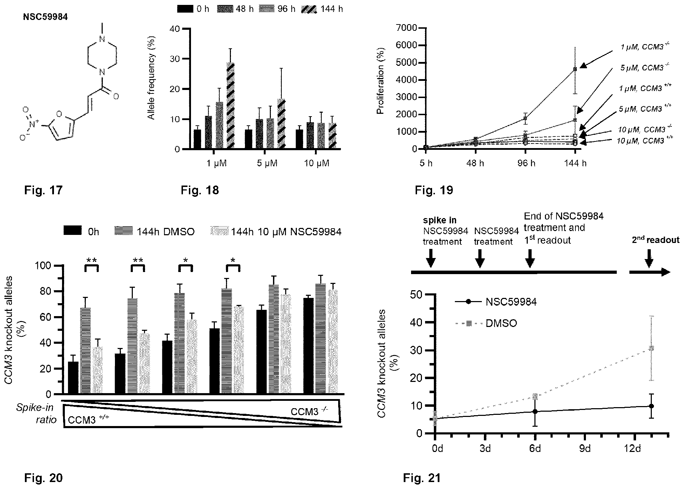

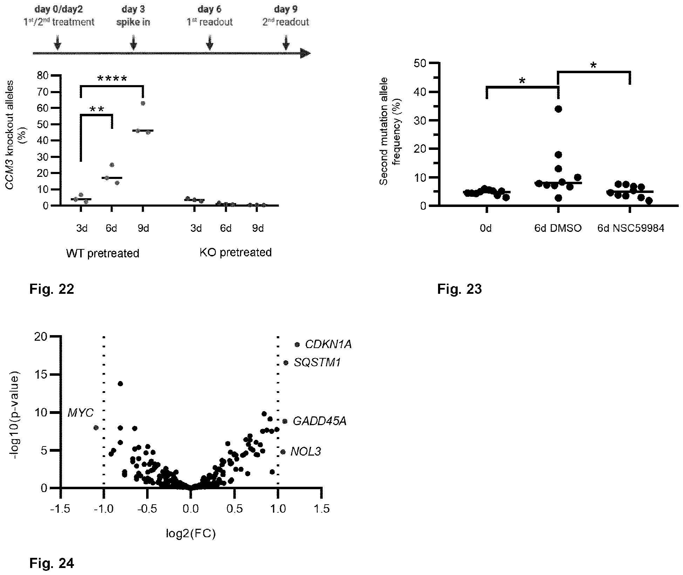

NSC59984 (compound of formula (Ia), see also Fig. 17) proved to be an extremely potent and selective inhibitor of mutant cell expansion. Additional co-culture experiments demonstrated that NSC59984 blocked the clonal expansion of *CCM3*^{*-*/*-*} CI-huVECs in a concentration-dependent manner and preferentially inhibited mutant EC expansion in co-culture with *CCM3*^{*+*/*+*} CI-huVECs (Fig. 18). The relative proliferation rate of *CCM3*^{*-*/*-*} CI-huVECs was reduced from 4632 % when cultured with only 1 µM NSC59984 to 1671 % when co-cultures were treated with 5 µM NSC59984 for six days. It further dropped to 419 % when NSC59984 was used at a concentration of 10 µM. On the other hand, the proliferation rate of wild-type ECs decreased to a lesser extent (761 % to 295 %, 10 µM, Fig. 19). Interestingly, NSC59984 blocked mutant EC expansion even at high spike-in ratios but did not decrease mutant allele frequencies (Fig. 20). These results are in line with the hypothesis that NSC59984 would trigger growth arrest rather than cell death in *CCM3*^{*-*/*-*} CI-huVECs. To determine the duration of the cytostatic activity, NSC59984 treatment was stopped after six days and measured the frequency of mutant alleles in co-culture after seven additional days without any treatment. The clonal expansion of mutant ECs was still efficiently blocked after the end of NSC59984 treatment (Fig. 21). Furthermore, pretreatment of *CCM3*^{*-*/*-*} CI-huVECs with NSC59984 before co-culture completely prevented mutant EC expansion and knockout alleles were hardly detectable in co-cultures after 6 days without additional treatment (Fig. 22). Finally, the question was whether NSC59984 was also useful for other genotypes and in primary ECs. NSC59984 significantly blocked the expansion of *CCM1*^{*-*/*-*} BOECs in co-culture with heterozygous *CCM1*^{*+*/*-*} BOECs (Fig. 23). Most interestingly, even clones with highly increased proliferation in co-culture were effectively inhibited by NSC59984 treatment.

Taken together, these results indicate that NSC59984 exhibited potent cytostatic activity and covers different genotypes.

### NSC59984 induced cell cycle arrest and induces upregulation of p53 target genes in mutant ECs

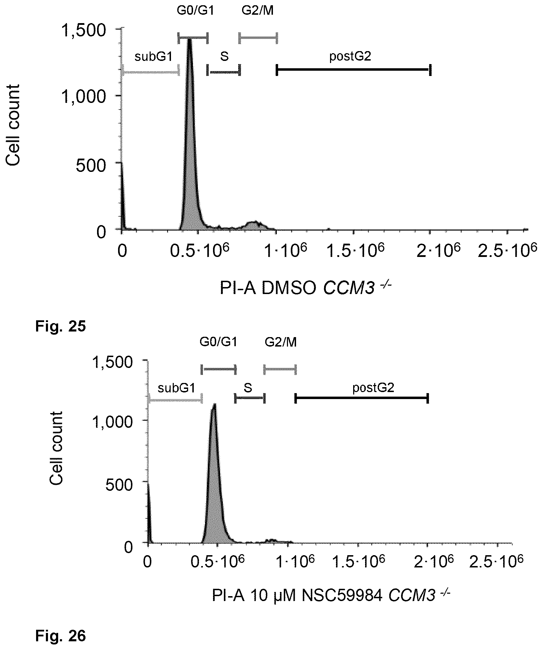

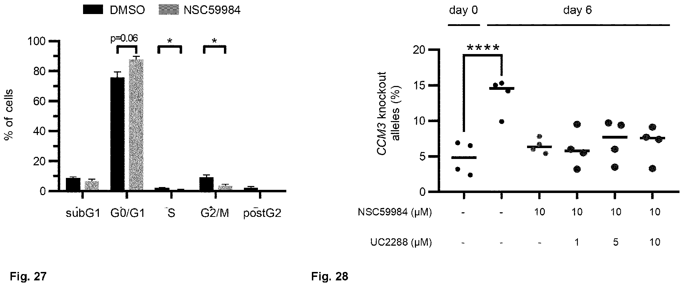

NSC59984 has exclusively been studied as p53 reactivator in the tumour context so far [9, 18]. To elucidate the mechanisms by which NSC59984 inhibits mutant EC proliferation, targeted RNA sequencing was used and the expression levels of genes associated with cell death and apoptosis in DMSO- and NSC59984-treated *CCM3*^{*-*/*-*} CI-huVECs were compared. NSC59984 increased the expression of *GADD45A* (FC = 2.11, p = 1.52x10⁻⁹) and *CDKN1A* (p21, FC = 2.33, p = 1.18x10⁻¹⁹) which encode for cyclin inhibitors and are known target genes of p53 (Fig. 24). In contrast, the expression of apoptosis-related p53 target genes, like *BBC3* (PUMA) or *PMAIP1* (Noxa), was unaffected (Fig. 24). To test whether NSC59984 acts by the induction of cell cycle arrest in *CCM3*^{*-*/*-*} CI-huVECs, the cell cycle profiles of DMSO- and NSC59984-treated mutant ECs were analysed. As expected, FACS results suggested G0/G1 arrest after NSC59984 treatment (87.7 % vs 75.9 %) (Fig. 25, Fig. 26, Fig. 27). The effect on clonal expansion of *CCM3*^{*-*/*-*} cells by treatment with NSC59984 (10 µM) could not be reversed by the specific p21 inhibitor UC2288 (1 to 10 µM) (n = 4 per group) (Fig. 28).

### NSC59984 exerted an anti-proliferative effect of CCM3^{-/}⁻ cells in hiPSC-derived BMEC co-cultures and vascular fusion organoids.

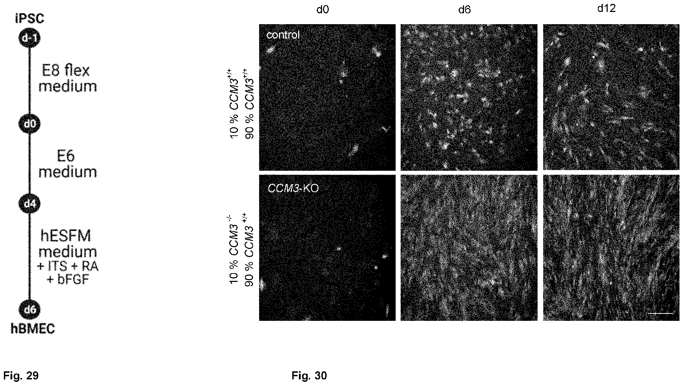

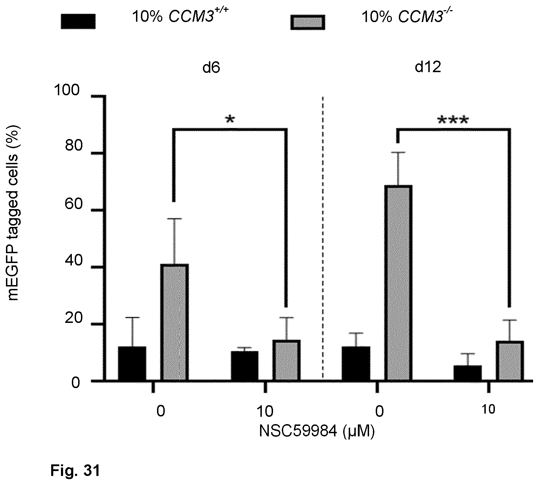

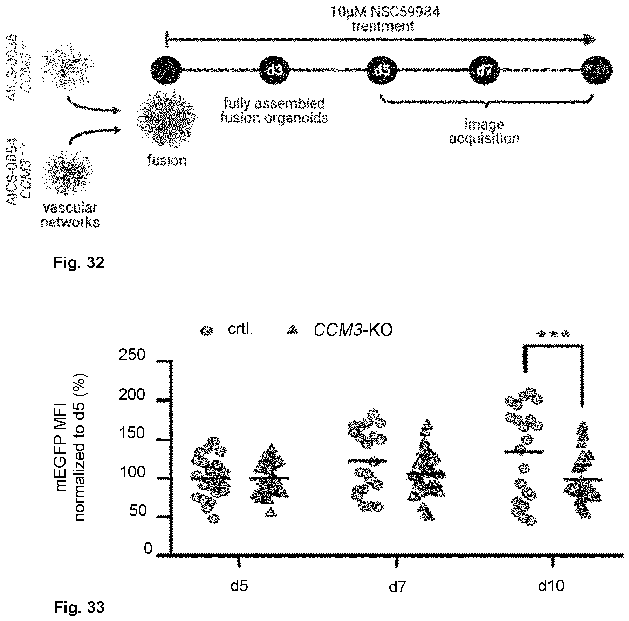

*CCM3*^{*-*/*-*} AICS-0036 (mEGFP), *CCM3*^{*+*/*+*} AICS-0036 (mEGFP), and *CCM3*^{*+*/*+*} AICS-0054 hiPCS were differentiated to brain microvascular endothelial-like cells (BMEC) (Fig. 29) and mixed in a 1:9 ratio (AICS-0036:AICS0054). In co-culture with *CCM3*^{*+*/*+*} BMECs, *CCM3*^{-/*-*}cells demonstrated a significant clonal expansion with a relative proportion of 69 % after 12 days (Fig. 30, Fig. 31). Additionally, co-cultures were treated with 10 µM NSC59984 for six days, released from NSC59984 treatment, and cultured for another six days under standard culture conditions. Even after release from treatment, NSC59984 blocked the clonal expansion of *CCM3*^{*-*/*-*} BMECs in co-culture (Fig. 31). In a next step, mEGFP-tagged *CCM3*^{+/+} or *CCM3*^{-/-}*(CCM3-KO)* hiPSC derived vascular networks were fused with mTagRFPT-tagged *CCM3*^{+/+} vascular networks and differentiated into fully encapsulated vascular fusion organoids in the presence of 10µM NSC9984. NSC59984 also inhibited the proliferation of *CCM3*^{-/-} cells in fusion organoids (Fig. 32, Fig. 33, Fig. 34).

### NSC59984 had a cytostatic effect on mutant ECs in co-culture.

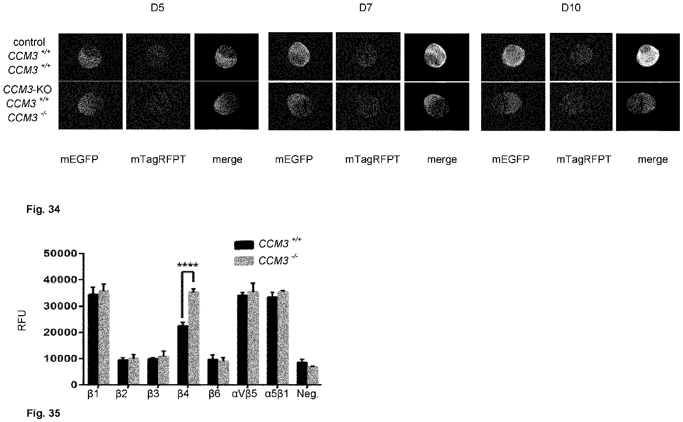

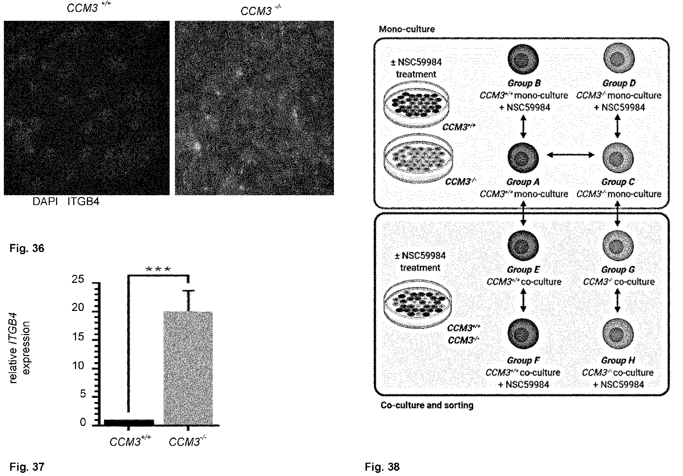

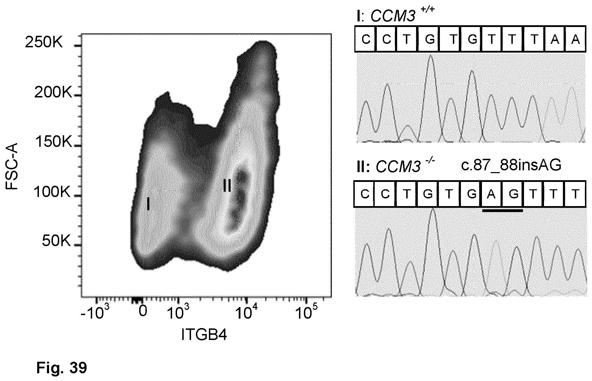

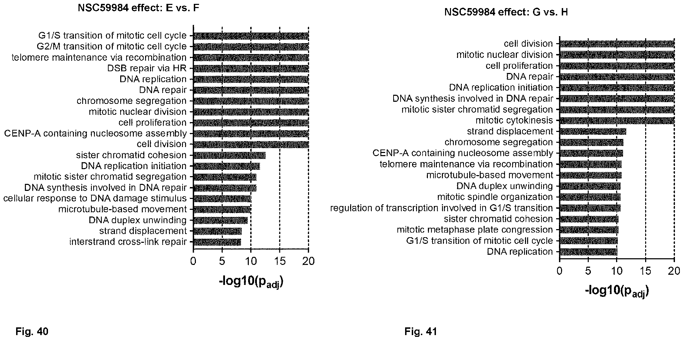

Using a β-integrin-mediated cell adhesion array of different integrin β subunits, a significantly increased surface expression of ITGB4 was observed on *CCM3*^{*-*/*-*} CI-huVECs (Fig. 35). In addition, immunofluorescence imaging and RT-qPCR analysis verified an upregulation of *ITGB4* gene expression in *CCM3*^{*-*/*-*} CI-huVECs (Fig. 36, Fig. 37). FACS sorting of co-cultured *CCM3*^{*-*/*-*} and *CCM3*^{*+*/*+*} CI-huVECs resulted in two distinct populations, and DNA sequencing verified high purities of sorted wild-type and mutant ECs, respectively (Fig. 39). Based on these results, we decided to set up a genome-wide gene expression profiling experiment. *CCM3*^{*+*/*+*} and *CCM3*^{*-*/*-*} CI-huVECs were co-cultured, treated with NSC59984 or DMSO control, sorted by FACS and analyzed by RNA sequencing. Treated and untreated *CCM3*^{*+*/*+*} and *CCM3*^{-/*-*}CI-huVEC monocultures served as controls (Fig. 38). Significantly up- or downregulated genes in NSC59984-treated co-cultures were subjected to gene ontology analysis. Notably, NSC59984 demonstrated similar effect on proliferation and cell cycle-related genes in *CCM3*^{*+*/*+*} and *CCM3*^{*-*/*-*} CI-huVECs (Figures 40, 41).

### DISCUSSION

Herein, it was demonstrated that the p53 pathway activator NSC59984 induced cell cycle arrest in *CCM3*^{*-*/*-*} ECs and inhibits their clonal expansion when co-cultured with wild-type ECs. Deregulation of the p53 pathway has not yet been described to be directly involved in CCM disease. However, genetic inactivation of *Trp53,* the mouse ortholog of *TP53,* is well known to sensitize *Ccm3*^{+/+} (aka *Pdcd10*^{*+*/*-*}), *Cem2*^{*+*/}*⁻,* and *Cem1*^{*+*/*-*} mice to CCM formation [19, 20]. The tumour protein p53 controls cell death, cellular senescence, cell cycle arrest, and other essential cellular processes. Deregulated signalling of this prototypical tumour suppressor contributes to several hallmarks of cancer. *TP53* mutations can be found in more than 40 % of all malignant tumours, and other mechanisms are thought to compromise p53 signalling in a significant proportion of the remaining cases [21, 22, 23]. CCM is not a malignant disease. However, the clinical phenotype of CCM, especially of patients with a *CCM3* mutation, can be rather aggressive, and deregulation along the p53 pathway provides a plausible explanation for various abnormalities observed after *CCM3* inactivation in human ECs. The role of CCM3 in apoptotic cell death is controversial but various studies have established that inactivation of *CCM3* can impair cellular sensitivity to apoptotic signals [7, 24]. The limitless proliferative potential and the abnormally high clonogenic capacity of *CCM3*^{*-*/*-*} ECs are additional features that are compatible with p53 malfunction.

Investigating the effects of a target gene knockout on p53 signalling *in vitro* can be challenging. Stable Cas9 expression has been shown to induce p53 pathway activation and selection for cells with p53-inactivating genetic variants [28]. Notably, the crRNA:tracrRNA:Cas9 RNP-mediated genome editing approach used in this study benefits from the transient nature of Cas9 expression. RNPs have a short intracellular half-life and are degraded within hours after transfection [29]. Combined with our spike-in assay, the cell culture model presented here closely recapitulates the situation *in vivo,* where a mosaic pattern of ECs with complete inactivation of a CCM protein and those with preserved CCM protein expression has been observed. Strikingly, only the direct co-culture of both cell types triggered an abnormal proliferation of mutant ECs. The tumour-like behaviour of *CCM3*^{*-*/*-*} cells in co-culture supports the hypothesis that the use of antiproliferative drugs is a reasonable approach to treat CCM disease. The observation that ponatinib, a kinase inhibitor with an antiproliferative activity that is used to treat chronic myeloid leukaemia and Ph⁺ acute lymphoblastic leukaemia, stopped CCM formation and lesion growth in *Ccm1^{iECKO}* mice [30] further substantiated this concept. However, ponatinib has a broad spectrum of side effects, e.g. thrombocytopenia, abdominal pain, anaemia, and even arrhythmia [31] which would likely limit its therapeutic use in CCM. Thus, the identification of further antiproliferative drug candidates is warranted. In a systemic screen of 189 small molecule compounds targeting apoptosis, survival, and proliferation, we identified NSC59984 as a potent inhibitor of mutant cell expansion. With minimal genotoxic effects on normal cells, NSC59984 has been shown to reduce cell growth in various human tumour cell lines by induction of MDM2-mediated mutant p53 protein degradation, activation of p73, and restoration of p53 signalling [9]. Without apparent adverse effects, NSC59984 also repressed the growth of colon-tumour xenografts in mice [9].

Consistent with previous observations in tumour cells, the upregulation of transcriptional target genes of p53, in particular *CDKN1A* (p21) and *GADD45A* (growth arrest and DNA-damage-inducible 45 alpha) [32], indicated activation of p53 signalling in *CCM3*^{*-*/*-*} ECs upon NSC59984 treatment. GADD45 and p21 are well-known to induce cell cycle arrest at G1/S and G2/M checkpoints [33, 34, 35]. Stopping the cell cycle progression of mutant ECs rather than directly killing them appears to be favourable in the CCM context because significant EC death would likely increase the haemorrhage risk of cavernomas. Interestingly, NSC59984 demonstrated potent anti-mutant effects *in vitro.* This small compound also effectively limited the proliferation of: (1) *CCM3*^{*-*/*-*} ECs co-cultured with wild-type ECs, (2) *CCM3*^{*-*/*-*} ECs co-cultured in high mutant-to-wild-type ratios, (3) *CCM1*^{*-*/*-*} BOECs co-cultured with heterozygous *CCM1*^{+/*-*}BOECs in a patient-specific *in vitro* model, (4) *CCM3*^{*-*/*-*} iPSC-derived human brain microvascular endothelial-like cells (hBMEC) co-cultured with wild-type iPSC-derived hBMEC, and (5) *CCM3*^{*-*/*-*} cells in iPSC-derived vascular organoids.

The presently used next-generation sequencing-based read-out system that allows tracking of mutant cell expansion on a genetic level will also facilitate testing of thousands of potential drug candidates. Such high-throughput screens may bring drug discovery in CCM research onto the next level.

In conclusion, the results indicate that malfunction of p53 signalling is involved in CCM disease. Although CCMs do not metastasise or grow invasively, the observations provide further evidence that mutant ECs have some cancer-like features that need to be targeted with novel CCM therapies. NSC59984 is a novel promising candidate drug that seems to be worth testing in further preclinical and clinical trials.

### Short description of the Figures

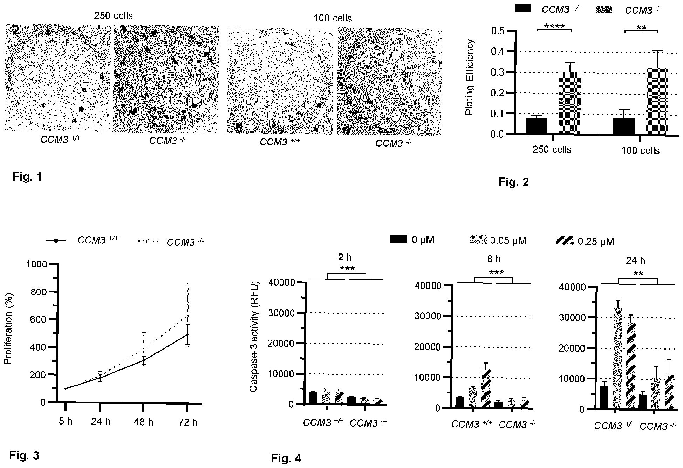

- **Fig. 1 to 11**: ***CCM3*^{*-*/*-*} CI-huVECs underwent clonal expansion only when co-cultured with *CCM3*^{+/+} ECs.**
- **Fig. 1**: *CCM3*^{*-*/*-*} and *CCM3*^{*+*/*+*} CI-huVECs were seeded in 6-well-plates with either 250 or 100 cells per well. Colonies were stained with crystal violet after eight days. Representative images are shown for both genotypes.
- **Fig. 2**: The plating efficiency of *CCM3*^{*-*/*-*} CI-huVECs was significantly increased under both seeding conditions (n = 4 per genotype).
- **Fig. 3**: *CCM3* inactivation did not significantly enhance proliferation of CI-huVECs under standard culture conditions (n = 9 per genotype).
- **Fig. 4**: CI-huVECs were treated with staurosporine (0.05 µM or 0.25 µM) to induce apoptotic cell death. After 2, 8, and 24 hours, the caspase-3 activity was markedly reduced in *CCM3*^{*-*/*-*} CI-huVECs (n = 3 per genotype).
- **Fig. 5**: A human apoptosis antibody array assay verified the reduction of active caspase-3 levels in staurosporine-treated (0.05 µM, 24 h) *CCM3*^{*-*/*-*} CI-huVECs (n = 3 per genotype). Representative array membranes are shown for both genotypes. Rectangles mark active caspase-3. No significant differences were found for other apoptosis markers.
- **Fig. 6**: The activities of caspase-8 and caspase-9 were also reduced in staurosporine-treated (0.05 µM or 0.25 µM, 8 h) *CCM3*^{*-*/*-*} CI-huVECs (n = 3 per genotype).
- **Fig. 7, 8**: The effect of *CCM3* gene disruption on the expression of apoptosis-related genes in human ECs was analysed by targeted RNA sequencing. *CCM3*^{*-*/*-*} and *CCM3*^{*+*/*+*} CI-huVECs were either treated with DMSO or staurosporine (0.05 µM, 24 h). The results are presented as volcano plots (n = 3 per group).
- **Fig. 9**: In a spike-in assay, *CCM3*^{*-*/*-*} CI-huVECs were co-cultured with *CCM3*^{*+*/*+*} CI--huVECs for six days. DNA was collected at indicated time points.
- **Fig. 10, 11**: The proportion of *CCM3* knockout alleles markedly increased over time and CCM3^{-/-} CI-huVECs proliferated significantly more in co-culture. RFU = relative fluorescence units. Data are presented as mean and SD. Students t test, two-way ANOVA or normalization analysis using the DESeq2 method were used for statistical analyses: **P<0.01; ***P<0.001; ****P<0.0001.
- **Fig. 12-16**: **NSC59984 efficiently blocked the clonal expansion of *CCM3*^{*-*/*-*} CI-huVECs in co-culture.**
- **Fig. 12**: Depicted is a scheme of the apoptosis library screening approach. Co-cultures of *CCM3*^{*-*/*-*} and *CCM3*^{*+*/*+*} CI-huVECs were treated with 10 µM of each library compound on day 0 and day 3. DNA samples were collected after six days. The frequencies of *CCM3* knockout alleles were determined with amplicon deep sequencing (ADS). DMSO treatment served as control.
- **Fig. 13**: NSC59984 and isoalantolactone reduced the portion of *CCM3* knockout alleles in co-culture samples significantly. In contrast, GSK-872 further enhanced the clonal expansion of *CCM3*^{*-*/*-*} CI-huVECs.
- **Fig. 14**: Bright-field microscopy demonstrated that cells treated with NSC59984 had a normal EC morphology while isoalantolactone treatment induced severe morphological changes and significant cell death. After GSK-872 treatment, cells had a compact morphology that is a known feature of *CCM3*^{*-*/*-*} CI-huVECs.

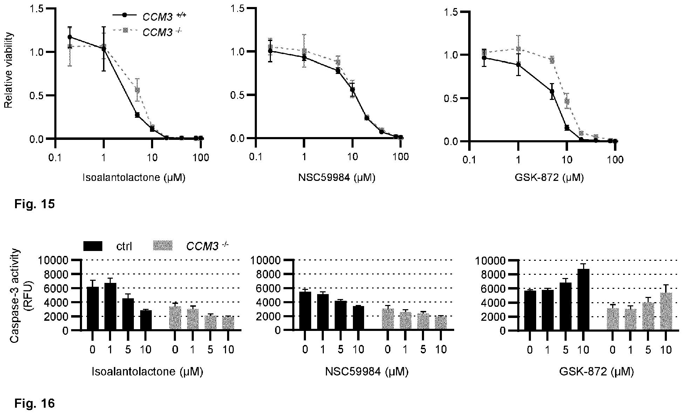

- **Fig. 15**: No cell viability differences were observed between *CCM3*^{*+*/*+*} and *CCM3*^{*-*/*-*} CI-huVECs 72 h after NSC59984 or isoalantolacone treatment while GSK-872 increased the viability of *CCM3*^{*-*/*-*} CI-huVECs.
- **Fig. 16**: NSC59984 and isoalantolactone treatment for 24 h did not increase caspase-3 activities in *CCM3*^{*+*/*+*} and *CCM3*^{*-*/*-*} CI-huVECs. Comp. = library compound. p_{adj}=adjusted p-value. RFU = relative fluorescence units. Data are presented as mean and SD (n = 3 per group). One-way ANOVA was used for statistical analyses.
- **Fig. 17-23**: **NSC59984 treatment predominantly affected *CCM3*^{*-*/*-*} ECs.**
- **Fig. 17**: Molecular structure of NSC59984 (CSID:4512175).
- **Fig. 18, 19**: NSC59984 blocked the clonal expansion of *CCM3*^{*-*/*-*} CI-huVECs in co-culture in a dose-dependent manner and mainly inhibited the proliferation of mutant ECs (n = 3 per group).
- **Fig. 20, 21**: NSC59984 (10 µM) reduced the clonal expansion of mutant ECs even at high spike-in ratios and retained its effect after treatment release (n = 3 per group).
- **Fig. 22**: Pre-treatment of *CCM3*^{*-*/*-*} rather than *CCM3*^{*+*/*+*} CI-huVECs before spike-in prevented an increase of *CCM3* knockout alleles in co-culture and demonstrated that NSC59984 affects predominantly mutant ECs (n = 3 per group).
- **Fig.23**: Clonal expansion of mutant ECs was also observed in an approach using a patient specific *CCM1*^{*-*/*-*} BOEC cell line. In co-culture with *CCM1*^{*+*/*-*} BOECs, a reduced increase of the second knockout allele indicated inhibition of clonal expansion by 10 µM NSC59984 (n = 10 per group). Data are presented as mean and SD. Multiple *t* test, Two-way ANOVA with Dunnett's multiple comparisons test and students *t* test were used for statistical analyses: *P<0.05; **P<0.01; ****P<0.0001.
- **Fig. 24-28**: **NSC59984 induced cell cycle arrest and p53 signalling in *CCM3*^{*-*/*-*} CI-huVECs.**
- **Fig. 24**: Targeted RNA sequencing demonstrated upregulation of p53 target genes in *CCM3*^{*-*/*-*} CI-huVECs after NSC59984 treatment (10 µM, 24 h). The results are presented as volcano plots (n = 3 per group). FC = fold change (NSC59984 vs. DMSO treatment).
- **Fig. 25, 26, 27**: Cell cycle analysis indicated G1 arrest in NSC59984-treated *CCM3*^{*-*/*-*} CI-huVECs (10 µM, 72 h). Representative flow cytometry histograms of DMSO- and NSC59984-treated mutant ECs are depicted in Fig.25, 26. Statistics are given in Fig. 27 (n = 3 per group).
- **Fig. 28**: The effect on clonal expansion of *CCM3*^{*-*/*-*} cells by treatment with NSC59984 (10 µM) could not be reversed by the specific p21 inhibitor UC2288 (1 to 10 µM) (n = 4 per group). Data are presented as mean and SD. Normalization analysis using the DESeq2 method, students *t* test and One-way ANOVA with uncorrected Fisher's LSD test were used for statistical analyses: *P<0.05; ****P<0.0001.
- **Fig. 29-34**: **NSC59984 exerted an anti-proliferative effect on *CCM3*^{*-*/*-*} cells in hiPSC-derived BMEC co-cultures and vascular fusion organoids.**
- **Fig. 29, 30, 31**: *CCM3*^{*-*/*-*} AICS-0036 (mEGFP), *CCM3*^{*+*/*+*} AICS-0036 (mEGFP), and *CCM3*^{+/+} AICS-0054 (mTagRFPT) hiPCS were differentiated to brain microvascular endothelial-like cells (BMEC) (Fig. 29) and mixed in a 1:9 ratio. *CCM3*^{*-*/*-*}BMECs demonstrated a significant clonal expansion in co-culture with *CCM3*^{*+*/*+*} BMECs, visible in the black/grey/white picture based on the increasing grey areas (Fig. 30, Fig. 31). Co-cultures were also treated with 10 µM NSC59984 for six days, released from NSC59984 treatment, and cultured for another six days under standard culture conditions. NSC59984 blocked the significant clonal expansion of *CCM3*^{-/-} BMECs in co-culture (Fig. 31, scale bar: 200 µm).
- **Fig. 32**: mEGFP-tagged *CCM3*^{*+*/*+*} (control) or *CCM3*^{*-*/}*⁻ (CCM3-KO)* hiPSC derived vascular networks were fused with mTagRFPT-tagged *CCM3*^{*+*/*+*} vascular networks and differentiated into fully encapsulated vascular fusion organoids in the presence of 10µM NSC9984. Fusion organoids were continuously cultured with NSC59984 and imaged on day 5, 7, and 10.
- **Fig. 33, 34**: NSC59984 inhibited the proliferation of *CCM3*^{*-*/*-*} cells in fusion organoids. Data from three independent biological replicates are presented in Fig. 33. MFI = mean fluorescence intensity. Representative images are shown in Fig. 34 (scale bar: 500 µm). Data are presented as mean and SD or as individual data points, respectively. Two-tailed t tests were used for statistical analyses: *P<0.05; ***P<0.001.
- **Fig. 35-41**: **NSC59984 had a cytostatic effect on mutant ECs in co-culture.**
- **Fig. 35**: A β-integrin-mediated cell adhesion array was used to analyse the surface expression of different integrin β subunits on *CCM3*^{*-*/*-*} CI-huVECs.
- **Fig. 36, 37**: Immunofluorescence imaging and RT-qPCR analysis verified upregulation of *ITGB4* gene expression in *CCM3*^{*-*/*-*} CI-huVECs (scale bar: 100 µm).
- **Fig. 38, 39**: Using high integrin beta 4 expression as marker for *CCM3*^{*-*/*-*} CI-huVECs, mutant ECs could be efficiently sorted from mutant/wild-type co-cultures by fluorescence activated cell sorting. Sanger sequencing of sorted ITGB4^{low} (I) and ITGB4^{high} (II) cell populations verified high purity of *CCM3*^{*+*/*+*} and *CCM3*^{*-*/*-*} CI-huVECs, respectively (Fig. 39). *CCM3*^{*+*/*+*} and *CCM3*^{*-*/*-*} CI-huVECs were co-cultured, treated with NSC59984 or DMSO control, sorted by FACS and analyzed by RNA sequencing. Treated and untreated *CCM3*^{*+*/*+*} and *CCM3*^{*-*/*-*} CI-huVEC monocultures served as controls (Fig. 38).
- **Fig. 40, 41**: Significantly up- or downregulated genes in NSC59984-treated co-cultures were also subjected to gene ontology analysis. Shown are the top 20 of significantly enriched biological process GO terms in wild-type (Fig. 40) and mutant ECs (Fig. 41) (n = 3 per group). Data are presented as mean and SD (n = 3 per group). Students t test, and Fisher exact test were used for statistical analyses: ***P<0.001; ****P<0.0001.

### Cited Literature

1. Batra S, Lin D, Recinos PF, Zhang J, Rigamonti D. Cavernous malformations: natural history, diagnosis and treatment. Nat Rev Neurol 2009, 5(12): 659-670.
2. Akers A, Al-Shahi Salman R, I AA, Dahlem K, Flemming K, Hart B, et al. Synopsis of Guidelines for the Clinical Management of Cerebral Cavernous Malformations: Consensus Recommendations Based on Systematic Literature Review by the Angioma Alliance Scientific Advisory Board Clinical Experts Panel. Neurosurgery 2017, 80(5): 665-680.
3. Spiegler S, Najm J, Liu J, Gkalympoudis S, Schroder W, Borck G, et al. High mutation detection rates in cerebral cavernous malformation upon stringent inclusion criteria: one-third of probands are minors. Mol Genet Genomic Med 2014, 2(2): 176-185.
4. McDonald DA, Shi C, Shenkar R, Gallione CJ, Akers AL, Li S, et al. Lesions from patients with sporadic cerebral cavernous malformations harbor somatic mutations in the CCM genes: evidence for a common biochemical pathway for CCM pathogenesis. Hum Mol Genet 2014, 23(16): 4357-4370.
5. Malinverno M, Maderna C, Abu Taha A, Corada M, Orsenigo F, Valentino M, et al. Endothelial cell clonal expansion in the development of cerebral cavernous malformations. Nat Commun 2019, 10(1): 2761.
6. Detter MR, Snellings DA, Marchuk DA. Cerebral Cavernous Malformations Develop Through Clonal Expansion of Mutant Endothelial Cells. Circ Res 2018, 123(10): 1143-1151.
7. Schwefel K, Spiegler S, Ameling S, Much CD, Pilz RA, Otto O, et al. Biallelic CCM3 mutations cause a clonogenic survival advantage and endothelial cell stiffening. J Cell Mol Med 2019, 23(3): 1771-1783.
8. Abdelilah-Seyfried S, Tournier-Lasserve E, Derry WB. Blocking Signalopathic Events to Treat Cerebral Cavernous Malformations. Trends Mol Med 2020, 26(9): 874-887.
9. Zhang S, Zhou L, Hong B, van den Heuvel AP, Prabhu VV, Warfel NA, et al. Small-Molecule NSC59984 Restores p53 Pathway Signaling and Antitumor Effects against Colorectal Cancer via p73 Activation and Degradation of Mutant p53. Cancer Res 2015, 75(18): 3842-3852.
10. Lopez-Ramirez MA, Fonseca G, Zeineddine HA, Girard R, Moore T, Pham A, et al. Thrombospondin1 (TSP1) replacement prevents cerebral cavernous malformations. J Exp Med 2017, 214(11): 3331-3346.
11. Zhai Y, Yu J, Iruela-Arispe L, Huang WQ, Wang Z, Hayes AJ, et al. Inhibition of angiogenesis and breast cancer xenograft tumor growth by VEGI, a novel cytokine of the TNF superfamily. Int J Cancer 1999, 82(1): 131-136.
12. Yang CR, Hsieh SL, Teng CM, Ho FM, Su WL, Lin WW. Soluble decoy receptor 3 induces angiogenesis by neutralization of TL1A, a cytokine belonging to tumor necrosis factor superfamily and exhibiting angiostatic action. Cancer Res 2004, 64(3): 1122-1129.
13. Bai C, Connolly B, Metzker ML, Hilliard CA, Liu X, Sandig V, et al. Overexpression of M68/DcR3 in human gastrointestinal tract tumors independent of gene amplification and its location in a four-gene cluster. Proc Natl Acad Sci U S A 2000, 97(3): 1230-1235.
14. Tsuji S, Hosotani R, Yonehara S, Masui T, Tulachan SS, Nakajima S, et al. Endogenous decoy receptor 3 blocks the growth inhibition signals mediated by Fas ligand in human pancreatic adenocarcinoma. Int J Cancer 2003, 106(1): 17-25.
15. Guerrero A, Iglesias C, Raguz S, Floridia E, Gil J, Pombo CM, et al. The cerebral cavernous malformation 3 gene is necessary for senescence induction. Aging Cell 2015, 14(2): 274-283.
16. Mandal P, Berger SB, Pillay S, Moriwaki K, Huang C, Guo H, et al. RIP3 induces apoptosis independent of pronecrotic kinase activity. Mol Cell 2014, 56(4): 481-495.
17. Spiegler S, Rath M, Much CD, Sendtner BS, Felbor U. Precise CCM1 gene correction and inactivation in patient-derived endothelial cells: Modeling Knudson's two-hit hypothesis in vitro. Mol Genet Genomic Med 2019: e755.
18. Tang F, Min L, Seebacher NA, Li X, Zhou Y, Hornicek FJ, et al. Targeting mutant TP53 as a potential therapeutic strategy for the treatment of osteosarcoma. J Orthop Res 2019, 37(3): 789-798.
19. Plummer NW, Gallione CJ, Srinivasan S, Zawistowski JS, Louis DN, Marchuk DA. Loss of p53 sensitizes mice with a mutation in Ccm1 (KRIT1) to development of cerebral vascular malformations. Am J Pathol 2004, 165(5): 1509-1518.
20. Shenkar R, Venkatasubramanian PN, Wyrwicz AM, Zhao JC, Shi C, Akers A, et al. Advanced magnetic resonance imaging of cerebral cavernous malformations: part II. Imaging of lesions in murine models. Neurosurgery 2008, 63(4): 790-797; discussion 797-798.
21. Kandoth C, McLellan MD, Vandin F, Ye K, Niu B, Lu C, et al. Mutational landscape and significance across 12 major cancer types. Nature 2013, 502(7471): 333-339.
22. Zhao Y, Aguilar A, Bernard D, Wang S. Small-molecule inhibitors of the MDM2-p53 protein-protein interaction (MDM2 Inhibitors) in clinical trials for cancer treatment. J Med Chem 2015, 58(3): 1038-1052.
23. Tovar C, Rosinski J, Filipovic Z, Higgins B, Kolinsky K, Hilton H, et al. Small-molecule MDM2 antagonists reveal aberrant p53 signaling in cancer: implications for therapy. Proc Natl Acad Sci U S A 2006, 103(6): 1888-1893.
24. Zhu Y, Wu Q, Xu JF, Miller D, Sandalcioglu IE, Zhang JM, et al. Differential angiogenesis function of CCM2 and CCM3 in cerebral cavernous malformations. Neurosurg Focus 2010, 29(3): E1.
25. Chen L, Tanriover G, Yano H, Friedlander R, Louvi A, Gunel M. Apoptotic functions of PDCD10/CCM3, the gene mutated in cerebral cavernous malformation 3. Stroke 2009, 40(4): 1474-1481.
26. Nickel AC, Wan XY, Saban DV, Weng YL, Zhang S, Keyvani K, et al. Loss of programmed cell death 10 activates tumor cells and leads to temozolomide-resistance in glioblastoma. J Neurooncol 2019, 141(1): 31-41.
27. Zhang H, Ma X, Deng X, Chen Y, Mo X, Zhang Y, et al. PDCD10 interacts with STK25 to accelerate cell apoptosis under oxidative stress. Front Biosci (Landmark Ed) 2012, 17: 2295-2305.
28. Enache OM, Rendo V, Abdusamad M, Lam D, Davison D, Pal S, et al. Cas9 activates the p53 pathway and selects for p53-inactivating mutations. Nat Genet 2020, 52(7): 662-668.
29. Kim S, Kim D, Cho SW, Kim J, Kim JS. Highly efficient RNA-guided genome editing in human cells via delivery of purified Cas9 ribonucleoproteins. Genome Res 2014, 24(6): 1012-1019.
30. Choi JP, Wang R, Yang X, Wang X, Wang L, Ting KK, et al. Ponatinib (AP24534) inhibits MEKK3-KLF signaling and prevents formation and progression of cerebral cavernous malformations. Sci Adv 2018, 4(11): eaau0731.
31. Chan O, Talati C, Isenalumhe L, Shams S, Nodzon L, Fradley M, et al. Side-effects profile and outcomes of ponatinib in the treatment of chronic myeloid leukemia. Blood Adv 2020, 4(3): 530-538.
32. Fischer M. Census and evaluation of p53 target genes. Oncogene 2017, 36(28): 3943-3956.
33. Tamura RE, de Vasconcellos JF, Sarkar D, Libermann TA, Fisher PB, Zerbini LF. GADD45 proteins: central players in tumorigenesis. Curr Mol Med 2012, 12(5): 634-651.
34. Abbas T, Dutta A. p21 in cancer: intricate networks and multiple activities. Nat Rev Cancer 2009, 9(6): 400-414.
35. Vogelstein B, Lane D, Levine AJ. Surfing the p53 network. Nature 2000, 408(6810): 307-310.
35. Lipps C, Klein F, Wahlicht T, Seiffert V, Butueva M, Zauers J, et al. Expansion of functional personalized cells with specific transgene combinations. Nat Commun 2018, 9(1): 994.
36. Schwefel K, Spiegler S, Much CD, Felbor U, Rath M. CRISPR/Cas9-mediated Generation of Human Endothelial Cell Knockout Models of CCM Disease. Methods Mol Biol 2020, 2152: 169-177.
37. Franken NA, Rodermond HM, Stap J, Haveman J, van Bree C. Clonogenic assay of cells in vitro. Nat Protoc 2006, 1(5): 2315-2319.
38. Schwefel K, Spiegler S, Kirchmaier BC, Dellweg PKE, Much CD, Pané-Farré J, et al. Fibronectin rescues aberrant phenotype of endothelial cells lacking either CCM1, CCM2 or CCM3. FASEB J 2020, 34(7): 9018-9033.
39. Wimmer RA, Leopoldi A, Aichinger M, Kerjaschki D, Penninger JM. Generation of blood vessel organoids from human pluripotent stem cells. Nat Protoc 2019.

WO 2016/053938 A1
US /2019298733 A1.
CN 110 693 886 A
WO 2019/115472 A1.

## Claims

1. A compound of formula (I) 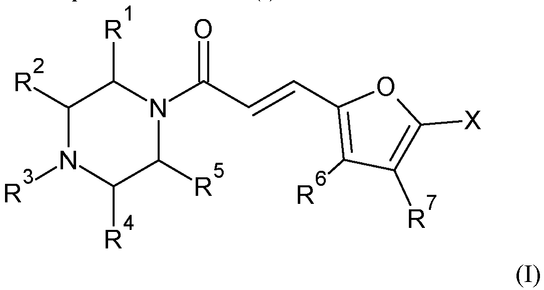 wherein
R¹, R², R⁴ and R⁵ are independently either a hydrogen atom or a straight or branched C1 to C3 alkyl group;
R³ is a hydrogen atom or a straight or branched C1 to C5 alkyl group;
R⁶, R⁷ are independently either a hydrogen atom or a straight or branched C1 to C3 alkyl group; and
X is selected from the group consisting of -F, -Cl, -Br, -I, -CN, -NO₂, -OR⁸, -SR⁸, -S(=O)R⁸, -S(=O)₂R⁵, -NHS(=O)₂R⁸, -C(=O)R⁸, -OC(=O)R⁸, -CO₂R⁸, -OCO₂R⁸, -CH(R⁸)₂, -N(R⁸)₂ -C(=O)N(R⁸)₂, -OC(=O)N(R⁸)₂, -NHC(=O)NH(R⁸)₂, -NHC(=O)R⁸, -NHC(=O)OR⁸, -C(OH) (R⁸)₂, and -C(NH₂)(R⁸)₂, wherein R⁸ is a hydrogen atom or a C1 to C3 alkyl group;
or a pharmaceutically acceptable salt thereof for use in treatment or prevention of a vascular malformation.

2. The compound of formula (I) or pharmaceutically acceptable salt thereof for use according to claim 1, wherein at least two, preferably at least three, of R¹, R², R⁴ and R⁵ are hydrogen atoms and the remaining residue of R¹, R², R⁴ and R⁵ is a straight or branched C1 to C3 alkyl group.

3. The compound of formula (I) or pharmaceutically acceptable salt thereof for use according to claim 1 or 2, wherein R³ is a straight or branched C1 to C5 alkyl group, preferably a straight or branched C1 to C3 alkyl group, more preferably an ethyl or methyl group, more preferably a methyl group.

4. The compound of formula (I) or pharmaceutically acceptable salt thereof for use according to any one of claims 1 to 3, wherein at least one of R⁶, R⁷ is a hydrogen atom, preferably R⁶ and R⁷ are both hydrogen atoms.

5. The compound of formula (I) or pharmaceutically acceptable salt thereof for use according to any one of claims 1 to 4, wherein X is selected from the group consisting of -CN, -NO₂, -C(=O)R⁸ -CO₂R⁸, -S(=O)R⁸ and -S(=O)₂R⁸, preferably from the group consisting of -CN, -NO₂, -C(=O)R⁸ -CO₂R⁸, wherein R⁸ is a hydrogen atom or a C1 to C3 alkyl group, wherein X is more preferably a -NO₂ group.

6. The compound of formula (I) or pharmaceutically acceptable salt thereof for use according to any one of claims 1 to 5, wherein the compound is NSC59984 of formula (Ia)

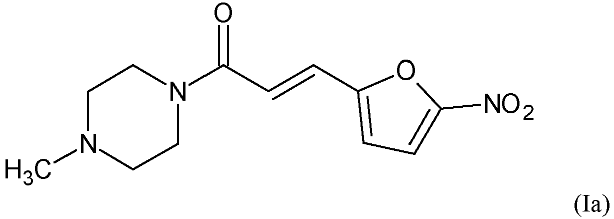

7. The compound or pharmaceutically acceptable salt thereof according to any one of claims 1 to 6 for use in treatment or prevention of vascular malformation, wherein the vascular malformation has a genetic component.

8. The compound or pharmaceutically acceptable salt thereof according to any one of claims 1 to 7 for use in treatment or prevention of vascular malformation, wherein the vascular malformation is selected from the group consisting of hereditary hemorrhagic telangiectasia, capillary malformation -arteriovenous malformation, glomuvenous malformations, infantile hemangioma, and cerebral cavernous malformation, wherein the vascular malformation is preferably cerebral cavernous malformation (CCM).

9. The compound according to any one of claims 1 to 8 for use in treatment or prevention of vascular malformation.

10. A method for identifying a subject profiting from treatment with a compound of formula (I) or pharmaceutically acceptable salt thereof according to any one of claims 1 to 9, comprising
(a) determining degree of vascular malformation in a sample of said subject;
(b) treating the sample with compound of formula (I) or pharmaceutically acceptable salt thereof according to any one of claims 1 to 9;
(c) determining degree of vascular malformation in a sample of said subject after (b);
(d) identifying a subject profiting from treatment with compound of formula (I) or pharmaceutically acceptable salt thereof according to any one of claims 1 to 9 in case the degree of vascular malformation is found to be reduced in (c).

11. The method of claim 10 which is an *in vitro* method, which is preferably based on proliferation of mutant endothelial cells (ECs).

## Patentansprüche

1. Verbindung der Formel (I) 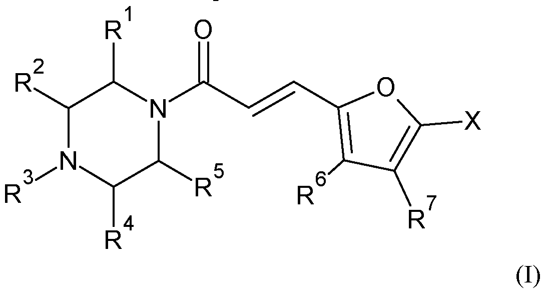 wobei
R¹, R², R⁴ und R⁵ unabhängig entweder ein Wasserstoffatom oder eine gerade oder verzweigte C1- bis C3-Alkylgruppe sind;
R³ ein Wasserstoffatom oder eine gerade oder verzweigte C1- bis C5-Alkylgruppe ist;
R⁶, R⁷ unabhängig entweder ein Wasserstoffatom oder eine gerade oder verzweigte C1- bis C3-Alkylgruppe sind; und
X ausgewählt ist aus der Gruppe bestehend aus -F, -Cl, -Br, -I, -CN, -NO₂, -OR⁸, -SR⁸, -S(=O)R⁸, -S(=O)₂R⁵, -NHS(=O)₂R⁸, -C(=O)R⁸, -OC(=O)R⁸, -CO₂R⁸, -OCO₂R⁸, -CH(R⁸)₂, -N(R⁸)₂ -C(=O)N(R⁸)₂, -OC(=O)N(R⁸)₂, -NHC(=O)NH(R⁸)₂, -NHC(=O)R⁸, -NHC(=O)OR⁸, -C(OH)(R⁸)₂ und -C(NH₂)(R⁸)₂, wobei R⁸ ein Wasserstoffatom oder eine C1- bis C3-Alkylgruppe ist;
oder ein pharmazeutisch unbedenkliches Salz davon zur Verwendung bei der Behandlung oder Prävention einer Gefäßfehlbildung.

2. Verbindung der Formel (I) oder pharmazeutisch unbedenkliches Salz davon zur Verwendung nach Anspruch 1, wobei mindestens zwei, vorzugsweise mindestens drei, von R¹, R², R⁴ und R⁵ Wasserstoffatome sind und der verbleibende Rest von R¹, R², R⁴ und R⁵ eine gerade oder verzweigte C1- bis C3-Alkylgruppe ist.

3. Verbindung der Formel (I) oder pharmazeutisch unbedenkliches Salz davon zur Verwendung nach Anspruch 1 oder 2, wobei R³ eine gerade oder verzweigte C1- bis C5-Alkylgruppe, vorzugsweise eine gerade oder verzweigte C1-bis C3-Alkylgruppe, weiter bevorzugt eine Ethyl- oder Methylgruppe, weiter bevorzugt eine Methylgruppe, ist.

4. Verbindung der Formel (I) oder pharmazeutisch unbedenkliches Salz davon zur Verwendung nach einem der Ansprüche 1 bis 3, wobei mindestens eines von R⁶, R⁷ ein Wasserstoffatom ist, vorzugsweise R⁶ und R⁷ beide Wasserstoffatome sind.

5. Verbindung der Formel (I) oder pharmazeutisch unbedenkliches Salz davon zur Verwendung nach einem der Ansprüche 1 bis 4, wobei X aus der Gruppe bestehend aus -CN, -NO₂, -C(=O)R⁸ -CO₂R⁸, -S(=O)R⁸ und -S(=O)₂R⁸, vorzugsweise aus der Gruppe bestehend aus -CN, -NO₂, -C(=O)R⁸ -CO₂R⁸, ausgewählt ist, wobei R⁸ ein Wasserstoffatom oder eine C1- bis C3-Alkylgruppe ist, wobei X weiter bevorzugt eine -NO₂-Gruppe ist.

6. Verbindung der Formel (I) oder pharmazeutisch unbedenkliches Salz davon zur Verwendung nach einem der Ansprüche 1 bis 5, wobei die Verbindung NSC59984 der Formel (Ia) ist:

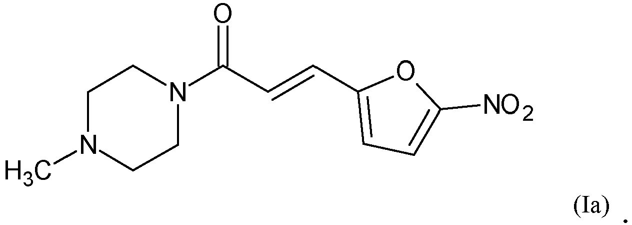

7. Verbindung oder pharmazeutisch unbedenkliches Salz davon nach einem der Ansprüche 1 bis 6 zur Verwendung bei der Behandlung oder Prävention von Gefäßfehlbildung, wobei die Gefäßfehlbildung eine genetische Komponente aufweist.

8. Verbindung oder pharmazeutisch unbedenkliches Salz davon nach einem der Ansprüche 1 bis 7 zur Verwendung bei der Behandlung oder Prävention von Gefäßfehlbildung, wobei die Gefäßfehlbildung aus der Gruppe bestehend aus hereditärer hämorrhagischer Telangiektasie, Kapillarfehlbildung-arteriovenöser Fehlbildung, glomuvenösen Fehlbildungen, infantilem Hämangiom und zerebraler kavernöser Fehlbildung ausgewählt ist, wobei die Gefäßfehlbildung vorzugsweise zerebrale kavernöse Fehlbildung (Cerebral Cavernous Malformation, CCM) ist.

9. Verbindung nach einem der Ansprüche 1 bis 8 zur Verwendung bei der Behandlung oder Prävention von Gefäßfehlbildung.

10. Verfahren zum Identifizieren eines Individuums, das von einer Behandlung mit einer Verbindung der Formel (I) oder einem pharmazeutisch unbedenklichen Salz davon nach einem der Ansprüche 1 bis 9 profitiert, umfassend
(a) Bestimmen des Grades der Gefäßfehlbildung in einer Probe des Individuums;
(b) Behandeln der Probe mit einer Verbindung der Formel (I) oder einem pharmazeutisch unbedenklichen Salz davon nach einem der Ansprüche 1 bis 9;
(c) Bestimmen des Grades der Gefäßfehlbildung in einer Probe des Individuums nach (b);
(d) Identifizieren eines Individuums, das von einer Behandlung mit Verbindung der Formel (I) oder einem pharmazeutisch unbedenklichen Salz davon nach einem der Ansprüche 1 bis 9 profitiert, falls festgestellt wird, dass der Grad der Gefäßfehlbildung in (c) verringert ist.

11. Verfahren nach Anspruch 10, welches ein *in-vitro-*Verfahren ist, das vorzugsweise auf der Proliferation von mutierten Endothelzellen (ECs) basiert.

## Revendications

1. Composé de formule (I) 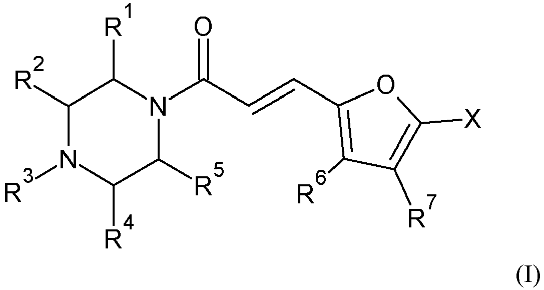
R¹, R², R⁴ et R⁵ étant indépendamment soit un atome d'hydrogène, soit un groupe alkyle en C1 à C3 linéaire ou ramifié ;
R³ étant un atome d'hydrogène ou un groupe alkyle en C1 à C5 linéaire ou ramifié ;
R⁶, R⁷ étant indépendamment soit un atome d'hydrogène, soit un groupe alkyle en C1 à C3 linéaire ou ramifié ; et
X étant choisi dans le groupe constitué par -F, -Cl, -Br, -I, -CN, -NO₂, -OR⁸, -SR⁸, -S(=O)R⁸, -S(=O)₂R⁵, -NHS(=O)₂R⁸, -C(=O)R⁸, -OC(=O)R⁸, -CO₂R⁸, -OCO₂R⁸, - CH(R⁸)₂, -N(R⁸)₂-C(=O)N(R⁸)₂, - OC(=O)N(R⁸)₂, -NHC (=O) NH (R⁸)₂, - NHC(=O)R⁸, -NHC(=O)OR⁸, -C(OH)(R⁸)₂, et -C(NH₂)(R⁸)₂, R⁸ étant un atome d'hydrogène ou un groupe alkyle en C1 à C3 ;
ou sel pharmaceutiquement acceptable de celui-ci pour une utilisation dans le traitement ou la prévention d'une malformation vasculaire.

2. Composé de formule (I) ou sel pharmaceutiquement acceptable de celui-ci pour une utilisation selon la revendication 1, au moins deux, de préférence au moins trois, parmi R¹, R², R⁴ et R⁵ étant des atomes d'hydrogène et le radical restant parmi R¹, R², R⁴ et R⁵ étant un groupe alkyle en C1 à C3 linéaire ou ramifié.

3. Composé de formule (I) ou sel pharmaceutiquement acceptable de celui-ci pour une utilisation selon la revendication 1 ou 2, R³ étant un groupe alkyle en C1 à C5 linéaire ou ramifié, de préférence un groupe alkyle en C1 à C3 linéaire ou ramifié, plus préférablement un groupe éthyle ou méthyle, plus préférablement un groupe méthyle.

4. Composé de formule (I) ou sel pharmaceutiquement acceptable de celui-ci pour une utilisation selon l'une quelconque des revendications 1 à 3, au moins l'un parmi R⁶, R⁷ étant un atome d'hydrogène, de préférence R⁶ et R⁷ étant tous deux des atomes d'hydrogène.

5. Composé de formule (I) ou sel pharmaceutiquement acceptable de celui-ci pour une utilisation selon l'une quelconque des revendications 1 à 4, X étant choisi dans le groupe constitué par -CN, -NO₂, -C(=O)R⁸ -CO₂R⁸, -S(=O)R⁸ et -S(=O)₂R⁸, de préférence dans le groupe constitué par -CN, -NO₂, -C(=O)R⁸ -CO₂R⁸, R⁸ étant un atome d'hydrogène ou un groupe alkyle en C1 à C3, X étant plus préférablement un groupe -NO₂.

6. Composé de formule (I) ou sel pharmaceutiquement acceptable de celui-ci pour une utilisation selon l'une quelconque des revendications 1 à 5, le composé étant NSC59984 de formule (Ia)

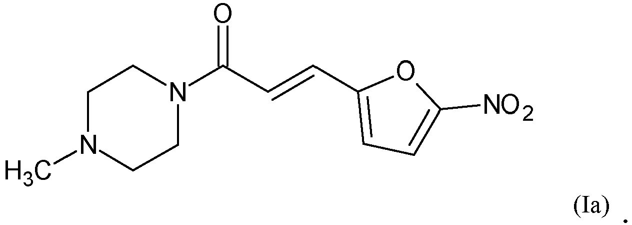

7. Composé ou sel pharmaceutiquement acceptable de celui-ci selon l'une quelconque des revendications 1 à 6, destiné à être utilisé dans le traitement ou la prévention d'une malformation vasculaire, la malformation vasculaire ayant un composant génétique.

8. Composé ou sel pharmaceutiquement acceptable de celui-ci selon l'une quelconque des revendications 1 à 7, destiné à être utilisé dans le traitement ou la prévention d'une malformation vasculaire, la malformation vasculaire étant choisie dans le groupe constitué par la télangiectasie hémorragique héréditaire, la malformation capillaire -la malformation artérioveineuse, les malformations glomuvénulaires, l'hémangiome infantile et la malformation cérébrale caverneuse, la malformation vasculaire étant de préférence une malformation cérébrale caverneuse (CCM).

9. Composé selon l'une quelconque des revendications 1 à 8, destiné à être utilisé dans le traitement ou la prévention d'une malformation vasculaire.

10. Procédé pour identifier un sujet profitant d'un traitement avec un composé de formule (I) ou un sel pharmaceutiquement acceptable de celui-ci selon l'une quelconque des revendications 1 à 9, comprenant
(a) la détermination du degré de malformation vasculaire dans un échantillon dudit sujet ;
(b) le traitement de l'échantillon avec un composé de formule (I) ou un sel pharmaceutiquement acceptable de celui-ci selon l'une quelconque des revendications 1 à 9 ;
(c) la détermination du degré de malformation vasculaire dans un échantillon dudit sujet après (b) ;
(d) l'identification d'un sujet profitant d'un traitement avec un composé de formule (I) ou un sel pharmaceutiquement acceptable de celui-ci selon l'une quelconque des revendications 1 à 9, dans le cas où le degré de malformation vasculaire s'avère réduit dans (c).

11. Procédé selon la revendication 10, qui est un procédé *in vitro,* qui est de préférence basé sur la prolifération de cellules endothéliales mutantes (EC).
